# EUROPEAN PATENT APPLICATION

(11) **EP 4 660 207 A1**
(43) Date of publication of application: **10.12.2025**
(21) Application number: 24750076.2
(22) Date of filing: 24.01.2024
(51) Int. Cl.: C08F 20/38, C08F 2/02, C08F 2/46, C08F 20/00, C08F 28/02, C08L 41/00, G02B 1/04, G02B 5/18

(54) **CURABLE COMPOSITION, CURED PRODUCT, OPTICAL MATERIAL, DIFFRACTIVE OPTICAL ELEMENT, AND COMPOUND**

(30) Priority: 31.01.2023 JP 2023012624; 28.07.2023 JP 2023123517; 17.01.2024 JP 2024005032
(71) Applicant: FUJIFILM Corporation, Tokyo 106-8620 (JP)
(72) Inventor: SHIRAIWA, Naozumi, Ashigarakami-gun, Kanagawa 258-8577 (JP); MOROOKA, Naoyuki, Ashigarakami-gun, Kanagawa 258-8577 (JP)
(74) Representative: HGF
(86) International application number: PCT/JP2024/001993
(87) International publication number: WO 2024/162126

(57) **Abstract**

Provided are a curable composition including a compound represented by General Formula (1) or (2), a cured product, an optical material, and a diffractive optical element, each of which uses the curable composition, and a compound represented by General Formula (1) or (2).

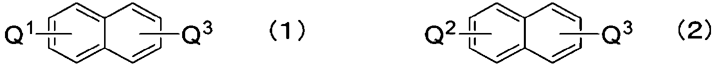

In the formulae, Q¹ represents a group represented by Formula (qla) or (qlb), Q² represents a group represented by Formula (q2), and Q³ represents a group represented by Formula (q3).

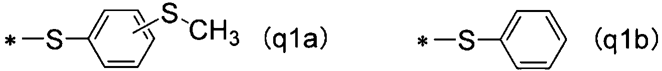

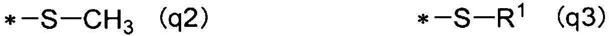

In the formulae, R¹ represents a (meth)acryloyl group, a vinyl group, or an allyl group. * represents a bonding site.

## Description

### BACKGROUND OF THE INVENTION

### 1. Field of the Invention

The present invention relates to a curable composition, a cured product, an optical material, a diffractive optical element, and a compound.

### 2. Description of the Related Art

In recent years, there have been active studies on optical resins that are expected to be applied to optical members such as diffractive optical elements, which require a high refractive index, and progress has also been made in the development of monomers that exhibit a high refractive index as raw materials for optical resins.

For example, JP2018-104696A describes a polymerizable compound represented by General Formula (1), having a specific structure in which a sulfur atom is bonded to a fused ring having a benzene ring and an aromatic ring.

### SUMMARY OF THE INVENTION

A nanofabrication technology called an imprinting technology, in which a mold having a microstructure with a variety of patterns having a size of nanometers to several hundred micrometers is embossed into a material such as a resin to transfer the patterns to a surface of the material, is known.

The imprinting consists of a step of applying a material onto a substrate, a step of pressing a mold in a state of being embossed, a step of fixing a transferred pattern by photocuring or thermal curing, and a step of demolding. The material used for imprinting is temporarily stored in a tank before use as in a case of an ink in ink jetting, before proceeding to the above-mentioned imprinting step, so it is required that crystals do not precipitate and turbidity does not occur in the storage tank (hereinafter, also referred to as "temporal stability").

An object of the present invention is to provide a curable composition which exhibits excellent temporal stability in a state of a curable composition before a curing reaction and which can also achieve an increase in refractive index of a cured product to be obtained. In addition, another object of the present invention is to provide a cured product obtained from the curable composition, an optical material and a diffractive optical element each including the cured product, and a compound contained in the curable composition.

The above-mentioned objects of the present invention have been achieved by the following means.
<1> A curable composition comprising: a compound represented by General Formula (1) or (2). 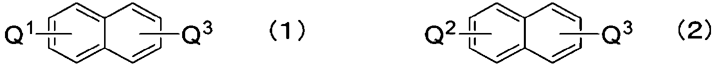 In the formulae, Q¹ represents a group represented by Formula (q1a) or (qlb), Q² represents a group represented by Formula (q2), and Q³ represents a group represented by Formula (q3). 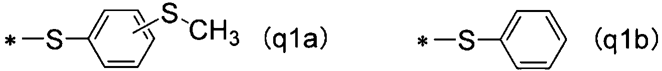 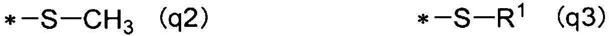 In the formulae, R¹ represents a (meth)acryloyl group, a vinyl group, or an allyl group. * represents a bonding site.
<2> The curable composition according to <1>, in which the compound represented by General Formula (1) or (2) includes at least one of a compound in which substitution positions of the substituents (Q¹ and Q³) in General Formula (1) are (1,5) positions, (1,6) positions, (2,7) positions, (5,1) positions, (6,1) positions, or (7,2) positions of a naphthalene ring, or a compound in which substitution positions of the substituents (Q² and Q³) in General Formula (2) are (1,5) positions, (1,6) positions, (2,7) positions, (5,1) positions, (6,1) positions, or (7,2) positions of a naphthalene ring.
<3> The curable composition according to <2>, in which the compound represented by General Formula (1) or (2) includes at least one compound represented by any of General Formulae (3) to (6). 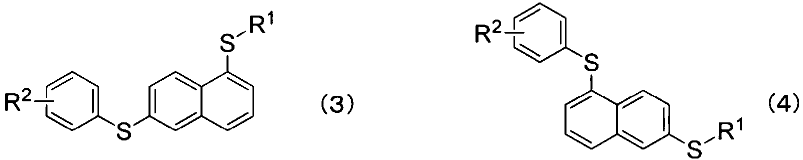 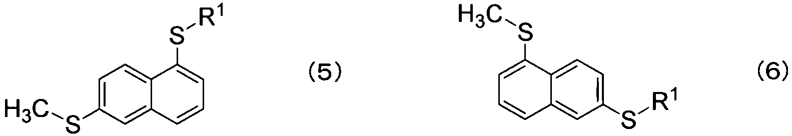 In the formulae, R¹ has the same definition as R¹ described above. R² represents a hydrogen atom or a methylsulfanyl group.
<4> The curable composition according to any one of <1> to <3>, further comprising: a compound represented by General Formula (7). 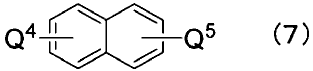 In the formula, Q⁴ and Q⁵ represent a group represented by any of Formula (qla), (qlb), (q2), or (q3). In this regard, one of Q⁴ and Q⁵ is a group represented by any of Formula (q1a), (qlb), or (q2) and the other is not a group represented by Formula (q3). 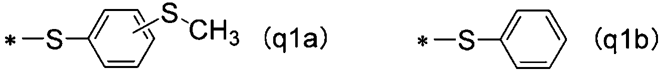 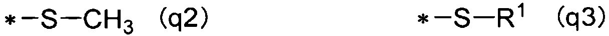 In the formulae, R¹ represents a (meth)acryloyl group, a vinyl group, or an allyl group. * represents a bonding site.
<5> The curable composition according to any one of <1> to <4>, in which the curable composition is for imprinting.
<6> A cured product obtained from the curable composition according to any one of <1> to <5>.
<7> An optical material comprising: the cured product according to <6>.
<8> A diffractive optical element which is formed of the cured product according to <6>, comprising: a surface having a diffraction grating shape.
<9> A compound represented by General Formula (1) or (2).

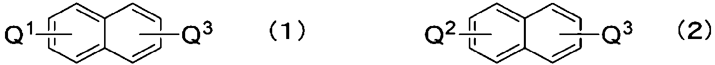

In the formulae, Q¹ represents a group represented by Formula (q1a) or (qlb), Q² represents a group represented by Formula (q2), and Q³ represents a group represented by Formula (q3).

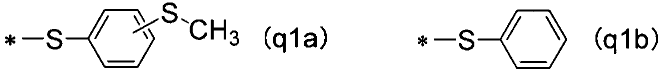

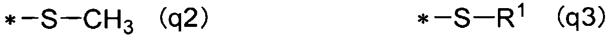

In the formulae, R¹ represents a (meth)acryloyl group, a vinyl group, or an allyl group. * represents a bonding site.

In the present invention, in a case where there are a plurality of substituents, linking groups, or the like (hereinafter, referred to as substituents or the like) represented by a specific reference numeral or formula, or in a case where a plurality of substituents or the like are defined simultaneously, unless otherwise specified, the substituents or the like may be the same as or different from each other (regardless of the presence or absence of the expression "each independently", the substituents or the like may be the same as or different from each other). The same applies to the definition of the number of substituents or the like. In a case where a plurality of substituents or the like come close to each other (particularly in a case where a plurality of substituents or the like are adjacent to each other), the substituents or the like may be linked to each other to form a ring, unless otherwise specified. In addition, unless otherwise specified, a ring, for example, an alicyclic ring, an aromatic ring, or a heterocyclic ring may be further condensed to form a fused ring.

In the present invention, unless otherwise specified, in a case where E and Z configurations for double bond are present in a molecule, the double bond may be any one thereof or may be a mixture thereof.

In addition, in the present invention, unless otherwise specified, in a case where a compound has one or two or more asymmetric carbons, for the stereochemistry of such asymmetric carbons, either an (R)-form or an (S)-form can be independently taken. As a result, the compound may be a mixture of stereoisomers such as optical isomers or diastereoisomers, or may be racemic.

In addition, in the present invention, the expression of the compound and the monomer is meant to include a compound or monomer in which the structure is partially modified within a range where the effect of the present invention is not impaired. Furthermore, the compound and the monomer that are not specifically described as substituted or unsubstituted are intended to mean that the compound or the monomer may have any substituent within a range where the effect of the present invention is not impaired. For example, in the compound represented by General Formula (1) or (2), the naphthalene ring having Q¹ and Q³ as substituents, the naphthalene ring having Q² and Q³ as substituents, and the benzene ring in the group represented by Formula (qla) or (qlb) may be unsubstituted or may have any substituent. Examples of any substituent include a saturated or unsaturated aliphatic hydrocarbon group which may be chain-like, branched, or cyclic, an aromatic hydrocarbon ring group, an aromatic heterocyclic group, an alkoxy group, an alkylsulfanyl group, an aryloxy group, an arylsulfanyl group, and a halogen atom. From the viewpoint of imprinting applications, it is preferable that the ring is unsubstituted, or in a case where the ring has a substituent, the substituent is a halogen atom.

In the present invention, with respect to a substituent that is not specified as substituted or unsubstituted (the same applies to a linking group and a ring), this means that the group may have any substituent within a range that does not impair the desired effect. For example, the term "alkyl group" means to include both an unsubstituted alkyl group and a substituted alkyl group.

In the present invention, in a case where the number of carbon atoms in a certain group is defined, the number of carbon atoms refers to the number of carbon atoms in the entire group unless otherwise specified in the present invention or the present specification. In other words, in a case where this group is in a form in which it further has a substituent, the number of carbon atoms refers to the total number of carbon atoms including the substituent.

In the present invention, any numerical range represented by using "to" refers to a range including numerical values described before and after "to" as a lower limit value and an upper limit value, respectively.

In the curable composition according to the embodiment of the present invention, each component may be used alone or in combination of two or more thereof. The same applies to a cured product, an optical material, and a diffractive optical element, which are obtained from the curable composition according to the embodiment of the present invention.

In the present invention, "(meth)acrylate" refers to either or both of acrylate and methacrylate, "(meth)acrylic acid" refers to either or both of acrylic acid and methacrylic acid and "(meth)acryloyl" refers to either or both of acryloyl and methacryloyl. In the present invention, a monomer is distinguished from an oligomer and a polymer based on the molecular weight, and a compound having a weight-average molecular weight of 1000 or less is referred to as the monomer.

In the present invention, the alkyl group refers to a linear or branched alkyl group.

The number of carbon atoms in the alkyl group is preferably 1 to 20, more preferably 1 to 10, still more preferably 1 to 7, and particularly preferably 1 to 5.

Examples of the alkyl group include a methyl group, an ethyl group, a propyl group, an isopropyl group, a butyl group, an isobutyl group, a sec-butyl group, a tert-butyl group, a pentyl group, a 1-methylbutyl group, a 3-methylbutyl group, a hexyl group, a 1-methylpentyl group, a 4-methylpentyl group, a heptyl group, a 1-methylhexyl group, a 5-methylhexyl group, a 2-ethylhexyl group, an octyl group, a 1-methylheptyl group, a nonyl group, a 1-methyloctyl group, a decyl group, an undecyl group, a dodecyl group, a tridecyl group, a tetradecyl group, a pentadecyl group, a hexadecyl group, a heptadecyl group, an octadecyl group, a nonadecyl group, and an eicosyl group, among which a methyl group or an ethyl group is preferable.

The same applies to an alkyl group in a group containing an alkyl group (an alkoxy group, an alkylsulfanyl group, or the like).

In addition, the alkyl group may have a substituent. Examples of such an alkyl group having a substituent include a halogenated alkyl group and a hydroxyalkyl group.

In the present invention, the alkylene group may be, for example, a group obtained by removing one hydrogen atom bonded to a carbon atom in the above-mentioned alkyl group. The alkylene group may be a linear alkylene group or a branched alkylene group. Examples of the alkylene group include an ethylene group, a propylene group, and a butylene group.

In the present invention, the monovalent aromatic hydrocarbon ring group (aryl group) refers to a monovalent group obtained by removing any one hydrogen atom from an aromatic hydrocarbon ring which may be a monocyclic ring or a fused ring. The monovalent aromatic hydrocarbon ring group is preferably an aromatic hydrocarbon ring group having 6 to 14 carbon atoms, and examples thereof include a phenyl group, a 1-naphthyl group, a 2-naphthyl group, a 1-anthracenyl group, a 2-anthracenyl group, a 9-anthracenyl group, a 1-phenanthryl group, a 2-phenanthryl group, a 3-phenanthryl group, a 4-phenanthryl group, and a 9-phenanthryl group. Among these groups, a phenyl group is more preferable.

The same applies to an aryl group in a group containing an aryl group (an aryloxy group, an arylsulfanyl group, or the like).

In the present invention, the monovalent aromatic heterocyclic group (heteroaryl group) represents a monovalent group obtained by removing any one hydrogen atom from an aromatic heterocyclic ring.

The aromatic heterocyclic ring refers to an aromatic ring where the ring is formed by carbon atoms and heteroatoms. Examples of the heteroatom include an oxygen atom, a nitrogen atom, and a sulfur atom. The aromatic heterocyclic ring may be a monocyclic ring or a fused ring and the number of atoms constituting the ring is preferably 5 to 20 and more preferably 5 to 14. The number of heteroatoms in the atoms constituting the ring is not particularly limited, and is preferably 1 to 3 and more preferably 1 or 2.

Examples of the heteroaryl group include a furyl group, a thienyl group, a pyrrolyl group, an imidazolyl group, an isothiazolyl group, an isoxazolyl group, a pyridyl group, a pyrazinyl group, a quinolyl group, a benzofuranyl group (preferably a 2-benzofuranyl group), a benzothiazolyl group (preferably a 2-benzothiazolyl group), and a benzoxazolyl group (preferably a 2-benzoxazolyl group).

In the present invention, examples of the halogen atom include a fluorine atom, a chlorine atom, a bromine atom, and an iodine atom.

The curable composition according to the embodiment of the present invention exhibits excellent temporal stability in a state before a curing reaction, and can also achieve a high refractive index of a cured product to be obtained. The cured product, optical material, and diffractive optical element according to the embodiment of the present invention can exhibit a high refractive index. The compound according to the embodiment of the present invention makes it possible to obtain the curable composition according to the embodiment of the present invention, which exhibits the above-mentioned excellent characteristics.

### BRIEF DESCRIPTION OF THE DRAWINGS

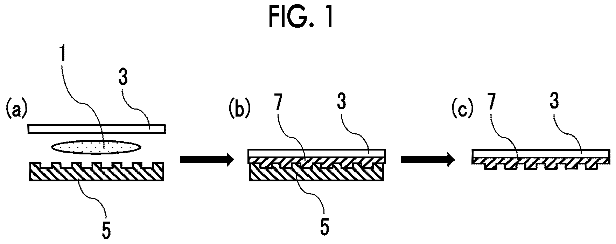

Fig. 1 is a schematic cross-sectional view schematically showing a method of manufacturing a cured product according to the embodiment of the present invention by an imprinting technology using a curable composition according to the embodiment of the present invention.

### DESCRIPTION OF THE PREFERRED EMBODIMENTS

### [Curable composition]

The curable composition according to the embodiment of the present invention contains a compound represented by General Formula (1) or (2) which will be described later.

In the present invention, the curable composition means a composition which has curing properties and which makes it possible to obtain a cured product (resin) by a curing reaction.

The compound represented by General Formula (1) or (2) which will be described later and which is contained in the curable composition according to the embodiment of the present invention is a polymerizable naphthalene sulfide compound which has a specific polymerizable group represented by R¹ through a sulfide bond on a naphthalene ring as represented by Q³, and further has a substituent having a specific structure through a sulfide bond as represented by Q¹ or Q².

The compound represented by General Formula (1) or (2) has the above-mentioned specific chemical structure, and thus exhibits excellent temporal stability in a state of a curable composition before a curing reaction in a case where the compound is made into the curable composition, and makes it possible to achieve a high refractive index of a cured product to be obtained at a high level.

Hereinafter, the components contained in the curable composition according to the embodiment of the present invention will be described in order.

### <Compound represented by General Formula (1) or (2)>

The curable composition according to the embodiment of the present invention contains a compound represented by General Formula (1) or (2).

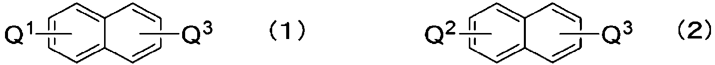

In the formulae, Q¹ represents a group represented by Formula (qla) or (qlb), Q² represents a group represented by Formula (q2), and Q³ represents a group represented by Formula (q3).

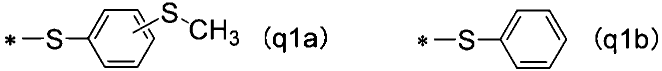

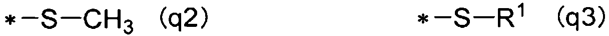

In the formulae, R¹ represents a (meth)acryloyl group, a vinyl group, or an allyl group. * represents a bonding site.

In the group represented by Formula (qla), a substitution position of the methylsulfanyl group (-SCH₃) on the benzene ring is not particularly limited, and the methylsulfanyl group may be bonded to any of the ortho position, the meta position, or the para position with respect to the bonding position of *-S- and is preferably bonded to the meta position.

From the viewpoint of further increasing a refractive index nD of liquid of the curable composition according to the embodiment of the present invention and a refractive index nD of the cured product according to the embodiment of the present invention, Q¹ is preferably a group represented by Formula (qla), and from the viewpoint of further lowering the viscosity of the composition, Q¹ is preferably a group represented by Formula (q1b).

R¹ is preferably a (meth)acryloyl group and more preferably an acryloyl group.

With regard to the compound represented by General Formula (1) or (2), from the viewpoint of further increasing the refractive index nD of liquid of the curable composition according to the embodiment of the present invention and the refractive index nD of the cured product according to the embodiment of the present invention, the compound represented by General Formula (1) is preferable, and from the viewpoint of further lowering the viscosity of the composition, the compound represented by General Formula (2) is preferable.

From the viewpoint of further increasing the refractive index nD of liquid and the refractive index nD of the cured product, the curable composition according to the embodiment of the present invention preferably contains the compound represented by General Formula (1), and from the viewpoint of further lowering the viscosity of the composition, the curable composition according to the embodiment of the present invention preferably contains the compound represented by General Formula (2).

The substitution positions of the substituents Q¹ and Q³ on the naphthalene ring in General Formula (1) may each be any of the 1- to 8-positions of the naphthalene ring.

The substitution positions of the substituents Q² and Q³ on the naphthalene ring in General Formula (2) may each be any of the 1- to 8-positions of the naphthalene ring.

In the present invention, the position numbers 1 to 8 of the naphthalene ring are as shown below.

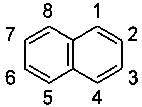

Examples of the substitution positions of the substituents (Q¹ and Q³) in General Formula (1) and the substitution positions of the substituents (Q² and Q³) in General Formula (2) include (1,5) positions, (1,6) positions, (2,6) positions, (2,7) positions, (5,1) positions, (6,1) positions, (6,2) positions, and (7,2) positions, among which (1,5) positions, (1,6) positions, (2,7) positions, (5,1) positions, (6,1) positions, or (7,2) positions are preferable.

The compound represented by General Formula (1) or (2) preferably includes at least one of a compound in which the substitution positions of the substituents (Q¹ and Q³) in General Formula (1) are the (1,5) positions, the (1,6) positions, the (2,7) positions, the (5,1) positions, the (6,1) positions, or the (7,2) positions of the naphthalene ring, or a compound in which the substitution positions of the substituents (Q² and Q³) in General Formula (2) are the (1,5) positions, the (1,6) positions, the (2,7) positions, the (5,1) positions, the (6,1) positions, or the (7,2) positions of the naphthalene ring.

In the present invention, the compound in which the substitution positions of the substituents (Q¹ and Q³) in General Formula (1) are the (1,5) positions means a compound in which the substitution position of Q¹ is the 1-position and the substitution position of Q³ is the 5-position, and the compound in which the substitution positions of the substituents (Q² and Q³) in General Formula (2) are the (1,5) positions means a compound in which the substitution position of Q² is the 1-position and the substitution position of Q³ is the 5-position. The same applies to the other (1,6) positions, (2,6) positions, (2,7) positions, (5,1) positions, (6,1) positions, (6,2) positions, and (7,2) positions.

The compound represented by General Formula (1) or (2) more preferably includes at least one of compounds represented by any of General Formulae (3) to (6).

Here, a compound represented by General Formula (3) and a compound represented by General Formula (5) correspond to a compound in which the substitution positions of the substituents (Q¹ and Q³) in General Formula (1) are the (6,1) positions and a compound in which the substitution positions of the substituents (Q² and Q³) in General Formula (2) are the (6,1) positions, respectively. In addition, a compound represented by General Formula (4) and a compound represented by General Formula (6) correspond to a compound in which the substitution positions of the substituents (Q¹ and Q³) in General Formula (1) are the positions (1,6) and a compound in which the substitution positions of the substituents (Q² and Q³) in General Formula (2) are the positions (1,6), respectively.

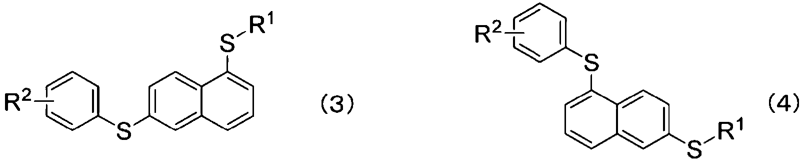

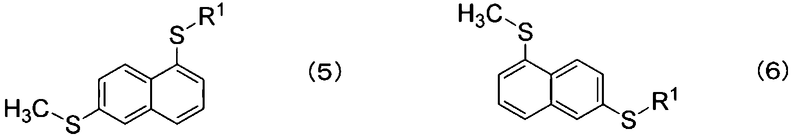

In the formulae, R¹ has the same definition as R¹ in General Formula (q3). R² represents a hydrogen atom or a methylsulfanyl group.

From the viewpoint of further increasing the refractive index nD of liquid of the curable composition according to the embodiment of the present invention and the refractive index nD of the cured product according to the embodiment of the present invention, R² is preferably a methylsulfanyl group, and from the viewpoint of further lowering the viscosity of the composition, R² is preferably a hydrogen atom.

In the compound represented by General Formula (1) or (2), the total content of the compound in which the substitution positions of the substituents (Q¹ and Q³) in General Formula (1) are the (1,5) positions, the (1,6) positions, the (2,7) positions, the (5,1) positions, the (6,1) positions, or the (7,2) positions of the naphthalene ring, and the compound in which the substitution positions of the substituents (Q² and Q³) in General Formula (2) are the (1,5) positions, the (1,6) positions, the (2,7) positions, the (5,1) positions, the (6,1) positions, or the (7,2) positions of the naphthalene ring is, for example, preferably 20% by mass or more, more preferably 50% by mass or more, still more preferably 65% by mass or more, and particularly preferably 80% by mass or more, and may be 100% by mass.

With regard to the "total content of the compounds represented by any of General Formulae (3) to (6)" in the compound represented by General Formula (1) or (2), the above description of "the total content of the compound in which the substitution positions of the substituents (Q¹ and Q³) in General Formula (1) are the (1,5) positions, the (1,6) positions, the (2,7) positions, the (5,1) positions, the (6,1) positions, or the (7,2) positions of the naphthalene ring, and the compound in which the substitution positions of the substituents (Q² and Q³) in General Formula (2) are the (1,5) positions, the (1,6) positions, the (2,7) positions, the (5,1) positions, the (6,1) positions, or the (7,2) positions of the naphthalene ring" can also be applied.

Hereinafter, preferred specific examples of the compound represented by General Formula (1) or (2) are listed, but the present invention is not limited to these compounds.

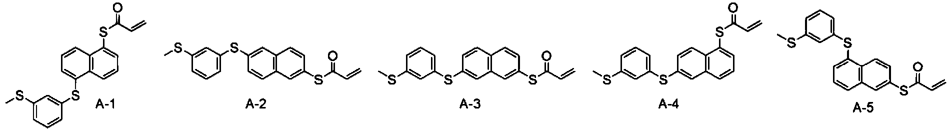

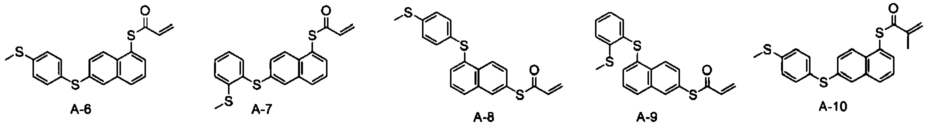

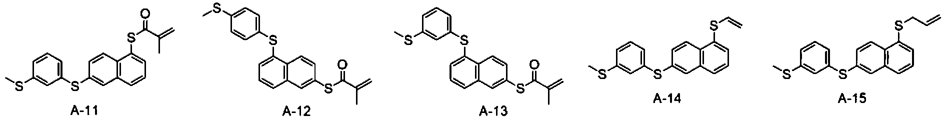

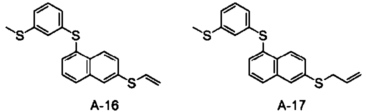

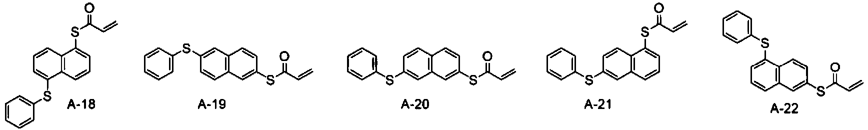

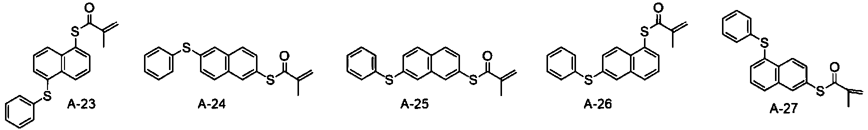

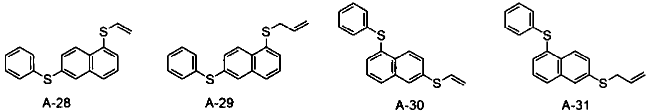

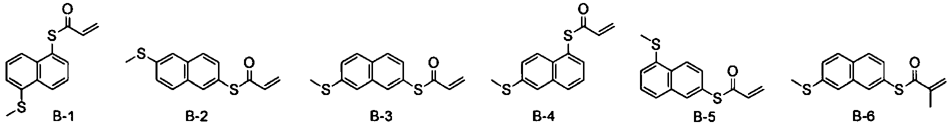

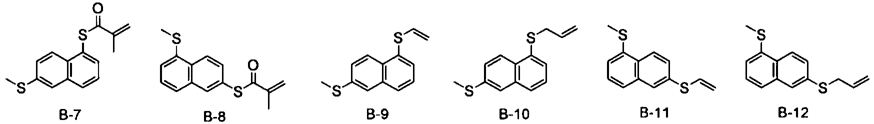

The molecular weight of the compound represented by General Formula (1) or (2) is preferably 232 to 600, more preferably 232 to 500, and still more preferably 232 to 400.

The compound represented by General Formula (1) or (2) can be synthesized by a common method. For example, the compound represented by General Formula (1) or (2) can be synthesized with reference to the synthesis method described in Org. Lett., 2004, Vol. 6, No. 24, pp. 4587 to 4590, the synthesis method described in US2003/0195270A, and the synthesis method described in J. Org. Chem., 2009, Vol. 74, No. 10, pp. 4005 to 4008. In addition, the compound represented by General Formula (1) or (2) can be synthesized as appropriate with reference to the methods described in Examples, and the like.

As described above, in a case where the compound represented by General Formula (1) or (2) is made into a curable composition containing the compound represented by General Formula (1) or (2), the obtained cured product can achieve a high refractive index. Moreover, even in a case where the concentration of the compound represented by General Formula (1) or (2) in the curable composition is set to a high concentration, the composition can exhibit excellent temporal stability.

In a case where the compound represented by General Formula (1) or (2) is made into the curable composition according to the embodiment of the present invention, which contains the compound represented by General Formula (1) or (2) at a high concentration, the content of the compound represented by General Formula (1) or (2) can be set to, for example, 99.99% by mass or less and is preferably 99.95% by mass or less, more preferably 99.90% by mass or less, and still more preferably 99.7% by mass or less. In this case, the lower limit value of the content of the compound represented by General Formula (1) or (2) can be set to, for example, 97% by mass or more and is preferably 98% by mass or more and more preferably 99.0% by mass or more. That is, the content of the compound represented by General Formula (1) or (2) can be set to 97% to 99.99% by mass and is preferably 98% to 99.95% by mass, more preferably 99.0% to 99.90% by mass, and still more preferably 99.0% to 99.7% by mass. In this case, the curable composition according to the embodiment of the present invention does not further contain a compound represented by General Formula (7) which will be described later.

In addition, in a case where the curable composition according to the embodiment of the present invention, which contains the compound represented by General Formula (1) or (2) at a high concentration, further contains the compound represented by General Formula (7) which will be described later as a compound contributing to a high refractive index, the total content of the compound represented by General Formula (1) or (2) and the compound represented by General Formula (7) can be set to, for example, 99.99% by mass or less and is preferably 99.95% by mass or less, more preferably 99.90% by mass or less, and still more preferably 99.7% by mass or less. In this case, the lower limit value of the total content of the compound represented by General Formula (1) or (2) and the compound represented by General Formula (7) can be set to, for example, 97% by mass or more and is preferably 98% by mass or more and more preferably 99.0% by mass or more. That is, the total content of the compound represented by General Formula (1) or (2) and the compound represented by General Formula (7) can be set to 97% to 99.99% by mass and is preferably 98% to 99.95% by mass, more preferably 99.0% to 99.90% by mass, and still more preferably 99.0% to 99.7% by mass.

The content of the compound represented by General Formula (1) or (2) in the total of the compound represented by General Formula (1) or (2) and the compound represented by General Formula (7) is not particularly limited and can be set to, for example, 10% to 90% by mass and is preferably 20% to 80% by mass, more preferably 25% to 75% by mass, and still more preferably 30% to 70% by mass. The content of the compound represented by General Formula (7) in the total of the compound represented by General Formula (1) or (2) and the compound represented by General Formula (7) is not particularly limited and can be set to, for example, 10% to 90% by mass and is preferably 20% to 80% by mass, more preferably 25% to 75% by mass, and still more preferably 30% to 70% by mass.

In a case where the curable composition according to the embodiment of the present invention contains a diluent monomer such as a difunctional (meth)acrylic acid thioester compound which will be described later, the content of the compound represented by General Formula (1) or (2) in the curable composition according to the embodiment of the present invention can be set to, for example, 60% by mass or more, and from the viewpoint of further improving (further increasing) the refractive index nD of liquid of the curable composition according to the embodiment of the present invention and the refractive index nD of the cured product according to the embodiment of the present invention (both of which are the refractive indexes at 25°C and a wavelength of 589 nm), the content of the compound represented by General Formula (1) or (2) in the curable composition according to the embodiment of the present invention is preferably 65% by mass or more, more preferably 70% by mass or more, and still more preferably 75% by mass or more. In this case, the upper limit value of the content of the compound represented by General Formula (1) or (2) is not particularly limited, and can be set to, for example, 99% by mass or less and is preferably 95% by mass or less, more preferably 90% by mass or less, and still more preferably 85% by mass or less. That is, the content of the compound represented by General Formula (1) or (2) is preferably 65% to 99% by mass, more preferably 70% to 95% by mass, still more preferably 75% to 90% by mass, and particularly preferably 75% to 85% by mass. In this case, the curable composition according to the embodiment of the present invention does not further contain a compound represented by General Formula (7) which will be described later.

In addition, in a case where the curable composition according to the embodiment of the present invention, which contains a diluent monomer such as a difunctional (meth)acrylic acid thioester compound which will be described later, further contains the compound represented by General Formula (7) which will be described later as a compound contributing to a high refractive index, the total content of the compound represented by General Formula (1) or (2) and the compound represented by General Formula (7) can be set to, for example, 60% by mass or more, and from the viewpoint of further improving (further increasing) the refractive index nD of liquid of the curable composition according to the embodiment of the present invention and the refractive index nD of the cured product according to the embodiment of the present invention (both of which are the refractive indexes at 25°C and a wavelength of 589 nm), the total content of the compound represented by General Formula (1) or (2) and the compound represented by General Formula (7) is preferably 65% by mass or more, more preferably 70% by mass or more, and still more preferably 75% by mass or more. In this case, the upper limit value of the total content of the compound represented by General Formula (1) or (2) and the compound represented by General Formula (7) is not particularly limited, and can be set to, for example, 99% by mass or less and is preferably 95% by mass or less, more preferably 90% by mass or less, and still more preferably 85% by mass or less. That is, the total content of the compound represented by General Formula (1) or (2) and the compound represented by General Formula (7) is preferably 65% to 99% by mass, more preferably 70% to 95% by mass, still more preferably 75% to 90% by mass, and particularly preferably 75% to 85% by mass.

The content of the compound represented by General Formula (1) or (2) in the total of the compound represented by General Formula (1) or (2) and the compound represented by General Formula (7) is not particularly limited and can be set to, for example, 10% to 90% by mass and is preferably 20% to 80% by mass, more preferably 25% to 75% by mass, and still more preferably 30% to 70% by mass. The content of the compound represented by General Formula (7) in the total of the compound represented by General Formula (1) or (2) and the compound represented by General Formula (7) is not particularly limited and can be set to, for example, 10% to 90% by mass and is preferably 20% to 80% by mass, more preferably 25% to 75% by mass, and still more preferably 30% to 70% by mass.

The curable composition according to the embodiment of the present invention may contain one type of the compound represented by General Formula (1) or (2), or may contain two or more types of the compounds represented by General Formula (1) or (2). That is, the curable composition according to the embodiment of the present invention contains at least one of the compounds represented by General Formula (1) or (2). In a case where at least one crystalline compound is used as the compound represented by General Formula (1) or (2), it is preferable that two or more types of the compounds represented by General Formula (1) or (2) are contained from the viewpoint of easily preparing a curable composition exhibiting excellent temporal stability. In a case where two or more types of compounds are contained, the combination thereof is not particularly limited, and the combination may be a combination of two or more types of only the compounds represented by General Formula (1), a combination of two or more types of only the compounds represented by General Formula (2), or a combination of one or more types of the compounds represented by General Formula (1) and one or more types of the compounds represented by General Formula (2).

The form in which the curable composition according to the embodiment of the present invention contains two or more types of the compounds represented by General Formula (1) or (2) may be, for example, a form in which a mixture of the compounds represented by General Formula (1) or (2) obtained by synthesis is contained.

In a case where two or more types of the compounds represented by General Formula (1) or (2) are contained, it is preferable that the total content thereof is within the range of the content of the compound represented by General Formula (1) or (2) described above. In a case where two or more types of the compounds represented by General Formula (7) are contained, it is preferable that the total content thereof is within the range of the content of the compound represented by General Formula (7) described above.

### <Compound represented by General Formula (7)>

It is also preferable that the curable composition according to the embodiment of the present invention further contains the compound represented by General Formula (7) in addition to the compound represented by General Formula (1) or (2) described above.

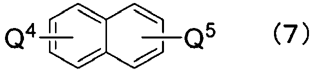

In the formula, Q⁴ and Q⁵ represent a group represented by any of Formula (qla), (q1b), (q2), or (q3) described above. In this regard, one of Q⁴ and Q⁵ is a group represented by any of Formula (qla), (qlb), or (q2) described above, and the other is not a group represented by Formula (q3) described above.

The combination of substituents that can be adopted as the substituents Q⁴ and Q⁵ is not particularly limited. For example, in the synthesis of the compound represented by General Formula (1) or (2) described above, the compound represented by General Formula (7) in which Q⁴ and Q⁵ are the same substituents is obtained as a by-product.

The substitution positions of the substituents Q⁴ and Q⁵ on the naphthalene ring in General Formula (7) may each be any of the 1- to 8-positions of the naphthalene ring.

The position numbers 1 to 8 of the naphthalene ring are as described above.

Examples of the substitution positions of the substituents (Q⁴ and Q⁵) in General Formula (7) include (1,5) positions, (1,6) positions, (2,6) positions, (2,7) positions, (5,1) positions, (6,1) positions, (6,2) positions, and (7,2) positions, among which (1,5) positions, (1,6) positions, (2,7) positions, (5,1) positions, (6,1) positions, or (7,2) positions are preferable.

The compound represented by General Formula (7) preferably includes a compound in which the substitution positions of the substituents (Q⁴ and Q⁵) are the (1,5) positions, the (1,6) positions, the (2,7) positions, the (5,1) positions, the (6,1) positions, or the (7,2) positions of the naphthalene ring, and more preferably includes a compound in which the substitution positions of the substituents (Q⁴ and Q⁵) are the (1,6) positions or the (6,1) positions of the naphthalene ring.

In the present invention, the compound in which the substitution positions of the substituents (Q⁴ and Q⁵) in General Formula (7) are the (1,5) positions means a compound in which the substitution position of Q⁴ is the 1-position and the substitution position of Q⁵ is the 5-position. The same applies to the other (1,6) positions, (2,6) positions, (2,7) positions, (5,1) positions, (6,1) positions, (6,2) positions, and (7,2) positions.

In the compound represented by General Formula (7), the total content of the compounds in which the substitution positions of the substituents (Q⁴ and Q⁵) in General Formula (7) are the (1,5) positions, the (1,6) positions, the (2,7) positions, the (5,1) positions, the (6,1) positions, or the (7,2) positions of the naphthalene ring is, for example, preferably 20% by mass or more, more preferably 50% by mass or more, still more preferably 65% by mass or more, and particularly preferably 80% by mass or more, and may be 100% by mass.

With regard to the "total content of the compounds in which the substitution positions of the substituents (Q⁴ and Q⁵) are the (1,6) positions or the (6,1) positions of the naphthalene ring" in the compound represented by General Formula (7), the above description of "the total content of the compounds in which the substitution positions of the substituents (Q⁴ and Q⁵) in General Formula (7) are the (1,5) positions, the (1,6) positions, the (2,7) positions, the (5,1) positions, the (6,1) positions, or the (7,2) positions of the naphthalene ring" can also be applied.

Hereinafter, preferred specific examples of the compound represented by General Formula (7) are listed, but the present invention is not limited to these compounds.

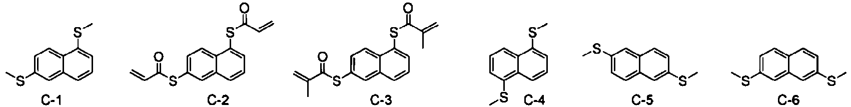

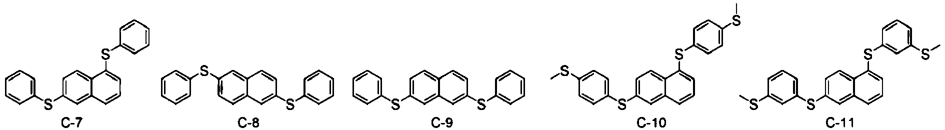

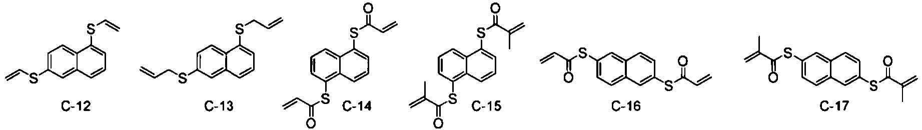

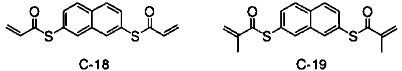

The molecular weight of the compound represented by General Formula (7) is preferably 220 to 600, more preferably 220 to 550, and still more preferably 220 to 500.

With regard to the method for synthesizing the compound represented by General Formula (7), the description pertaining to the method for synthesizing the compound represented by General Formula (1) or (2) can be appropriately cited and applied.

In the curable composition according to the embodiment of the present invention, the total content of the compound represented by General Formula (1) or (2) and the compound represented by General Formula (7), and the content of the compound represented by General Formula (7) in the total of the compound represented by General Formula (1) or (2) and the compound represented by General Formula (7) are as described above.

In a case where the curable composition according to the embodiment of the present invention further contains the compound represented by General Formula (7) in addition to the compound represented by General Formula (1) or (2), the compound represented by General Formula (1) or (2) contained may be one or more types or may be two or more types, and the compound represented by General Formula (7) contained may be one or more types or may be two or more types.

In a case where two or more types of the compounds represented by General Formula (1) or (2) are contained, the combination thereof is not particularly limited, and the combination may be a combination of two or more types of only the compounds represented by General Formula (1), a combination of two or more types of only the compounds represented by General Formula (2), or a combination of one or more types of the compounds represented by General Formula (1) and one or more types of the compounds represented by General Formula (2).

The compound represented by General Formula (1) or (2) includes both a liquid (non-crystalline) compound and a crystalline compound.

In a case where the compound represented by General Formula (1) or (2) is a crystalline compound, it is preferable to use a mixture of two or more types of the compounds represented by General Formula (1) or (2) or a mixture of two or more types of compounds in total, including one or more types of the compounds represented by General Formula (1) or (2) and one or more types of the compounds represented by General Formula (7) from the viewpoint of suppressing crystal precipitation of the compound, and it is more preferable to use a mixture of three or more types of the compounds represented by General Formula (1) or (2) or a mixture of three or more types of compounds in total, including one or more types of the compounds represented by General Formula (1) or (2) and one or more types of the compounds represented by General Formula (7) from the viewpoint of further suppressing crystal precipitation of the compound.

The above-mentioned form in which a total of three or more types of compounds are contained is not particularly limited, and examples thereof include a form in which a mixture of the compound represented by General Formula (1), the compound represented by General Formula (2), and the compound represented by General Formula (7), which are obtained by synthesis, is contained.

As described above, in a case where the compound represented by General Formula (1) or (2) is a liquid compound, the compound can be used as it is, and in a case where the compound represented by General Formula (1) or (2) is a crystalline compound, the compound can be used as the above-mentioned mixture of two or more types of compounds (preferably the above-mentioned mixture of three or more types of compounds), which allows the compound to be handled as a compound in which crystal precipitation is suppressed, and provides excellent handleability in a case of preparing a curable composition, and also allows the prepared curable composition to be suppressed from crystal precipitation.

In addition, the compound represented by General Formula (1) or (2) includes both a compound with almost no coloration and a compound with slight coloration.

Even in a case where the compound represented by General Formula (1) or (2) is a compound with slight coloration, the compound can be handled as a compound with almost no coloration in addition to the suppression of crystal precipitation by using the compound as the above-mentioned mixture of two or more types of compounds (preferably the above-mentioned mixture of three or more types of compounds).

The coloration of the compound represented by General Formula (1) or (2) can be evaluated by the transmittance at a wavelength of 450 nm. Therefore, the above-mentioned "with slight coloration" means that the transmittance at a wavelength of 450 nm is 91% or more and less than 96%, and the above-mentioned "with almost no coloration" means that the transmittance at a wavelength of 450 nm is 96% or more.

The "transmittance of the compound represented by General Formula (1) or (2) at a wavelength of 450 nm" is a value measured by spreading a melted sample to a thickness of 150 µm and using an ultraviolet-visible spectrophotometer (for example, UV-2600 (trade name), manufactured by Shimadzu Corporation), and the measurement conditions are as described in Examples which will be described later.

### <Other components>

The curable composition according to the embodiment of the present invention may further contain other components in addition to the compound represented by General Formula (1) or (2) and the compound represented by General Formula (7) which may be contained. Examples of other components include a difunctional (meth)acrylic acid thioester compound and a photoradical polymerization initiator.

### (Difunctional (meth)acrylic acid thioester compound)

The curable composition according to the embodiment of the present invention preferably contains, as a monomer for dilution, a difunctional (meth)acrylic acid thioester compound in addition to the above-mentioned compound represented by General Formula (1) or (2) and the above-mentioned compound represented by General Formula (7) which may be contained.

The difunctional (meth)acrylic acid thioester compound means a compound having two (meth)acryloylthio groups.

The group that links two (meth)acryloylthio groups (hereinafter, referred to as "linking group LL") is not particularly limited, and preferably does not have a branched structure and is, for example, more preferably a linear alkylene group or a group in which, one -CH₂- or the -CH₂- not adjacent to an S atom among two or more non-adjacent -CH₂-'s in a linear alkylene group is substituted with -S-.

The number of carbon atoms in the linear alkylene group that can be taken as the linking group LL is preferably 1 to 9, more preferably 3 to 7, and still more preferably 3 to 5.

In the present invention, the "group in which, one -CH₂- or the -CH₂- not adjacent to an S atom among two or more non-adjacent -CH₂-'s in a linear alkylene group is substituted with -S-" means, in other words, a group which has an -S- bond in a linear alkylene group, which does not have a structure in which two or more S atoms are linked (for example, a disulfide structure or a polysulfide structure in which three or more S atoms are linked), and in which a bonding site to a (meth)acryloylthio group is -CH₂-.

In the group in which, one -CH₂- or the -CH₂- not adjacent to an S atom among two or more non-adjacent -CH₂-'s in a linear alkylene group is substituted with -S-, the description of the linear alkylene group that can be taken as the linking group LL can be preferably applied to the linear alkylene group before -CH₂- is substituted with -S-.

The molecular weight of the difunctional (meth)acrylic acid thioester compound is preferably 150 to 400 and more preferably 200 to 350.

Hereinafter, preferred specific examples of the difunctional (meth)acrylic acid thioester compound are listed, but the present invention is not limited to these compounds.

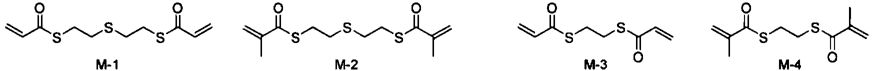

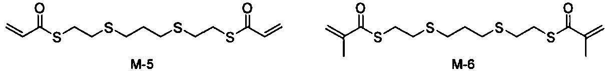

Among these compounds, the above-mentioned exemplary compound M-1 or M-2 is preferable as the difunctional (meth)acrylic acid thioester compound.

The method for obtaining the above-mentioned difunctional (meth)acrylic acid thioester compound is not particularly limited, and the difunctional (meth)acrylic acid thioester compound may be commercially available or may be synthesized by a common method.

In a case where the curable composition according to the embodiment of the present invention contains the difunctional (meth)acrylic acid thioester compound, the content of the difunctional (meth)acrylic acid thioester compound in the curable composition according to the embodiment of the present invention can be set to, for example, 40% by mass or less and is preferably 35% by mass or less, more preferably 30% by mass or less, and still more preferably 25% by mass or less. The lower limit value of the content of the difunctional (meth)acrylic acid thioester compound is not particularly limited and can be set to, for example, 1% by mass or more and is preferably 5% by mass or more, more preferably 10% by mass or more, and still more preferably 15% by mass or more. That is, the content of the difunctional (meth)acrylic acid thioester compound can be set to 1% to 40% by mass and is preferably 5% to 35% by mass, more preferably 10% to 30% by mass, and still more preferably 15% to 25% by mass.

By controlling the amount of the difunctional (meth)acrylic acid thioester compound in the curable composition, it is possible to adjust the function of relieving stress in a case where the cured product undergoes thermal changes.

In a case where the curable composition according to the embodiment of the present invention contains a difunctional (meth)acrylic acid thioester compound, the curable composition according to the embodiment of the present invention may contain two or more types of difunctional (meth)acrylic acid thioester compounds. In a case where two or more types of difunctional (meth)acrylic acid thioester compounds are contained, the total content of the two or more types of difunctional (meth)acrylic acid thioester compounds is preferably within the above-mentioned range.

### (Photoradical polymerization initiator)

The curable composition according to the embodiment of the present invention preferably contains a photoradical polymerization initiator. The curable composition according to the embodiment of the present invention makes it possible to obtain a cured product exhibiting a high refractive index by photopolymerization due to the action of the photoradical polymerization initiator. As the photoradical polymerization initiator, a compound that is commonly used as a photoradical polymerization initiator can be appropriately used according to the conditions of a photopolymerization (photocuring) step which will be described later, and specifically, the following compounds can be used.

Examples of the photoradical polymerization initiator include 1,2-diphenylethanedione, methylphenylglyoxylate; α-acylphosphine oxide compounds such as bis(2,6-dimethoxybenzoyl)-2,4,4-trimethylpentylphosphine oxide, bis(2,6-dimethylbenzoyl)-2,4,4-trimethylpentylphosphine oxide, bis(2,4,6-trimethylbenzoyl)-2,4,4-trimethylpentylphosphine oxide, bis(2,6-dichlorobenzoyl)-2,4,4-trimethylpentylphosphine oxide, (2,4,6-trimethylbenzoyl)diphenylphosphine oxide, bis(2,4,6-trimethylbenzoyl)phenylphosphine oxide, and ethylphenyl(2,4,6-trimethylbenzoyl)phosphinate (also known as (2,4,6-trimethylbenzoyl)ethoxylphenylphosphine oxide); α-hydroxyketone compounds such as 1-phenyl-2-hydroxy-2-methylpropan-1-one, 1-hydroxycyclohexylphenylketone,1-(4-isopropylphenyl)-2-hydroxy-2-methylpropan-1-one, 1-[4-(2-hydroxyethoxy)-phenyl]-2-hydroxy-2-methylpropan-1-one, and 2-hydroxy-1-{4-[4-(2-hydroxy-2-methyl-propionyl)-benzyl]phenyl}-2-methylpropan-1-one; benzyl ketal compounds such as 2,2-dimethoxy-1,2-diphenylethan-1-one; α-aminoketone compounds such as 2-methyl-1-(4-methylthiophenyl)-2-morpholinopropan-1-one and 2-benzyl-2-dimethylamino-1-(4-morpholinophenyl)-1-butanone; and oxime ester compounds such as 1-[4-(phenylthio)phenyl]octane-1,2-dione=2-(O-benzoyloxime) (IRGACURE OXE01 (trade name), available from BASF Japan Ltd.), 1-[9-ethyl-6-(2-methylbenzoyl)-9H-carbazol-3-yl]ethanone=O-acetyloxime (IRGACURE OXE02 (trade name), available from BASF Japan Ltd.), IRGACURE OXE03 and IRGACURE OXE04 (both of which are trade names, available from BASF Japan Ltd.), and ADEKA ARKLS N-1919T, ADEKA ARKLS NCI-831E, ADEKA ARKLS NCI-930, and ADEKA ARKLS NCI-730 (all of which are trade names, available from ADEKA Corporation).

Among these, in the present invention, 1-hydroxycyclohexyl phenyl ketone (IRGACURE 184 (trade name), available from BASF Japan Ltd.), bis(2,4,6-trimethylbenzoyl)phenylphosphine oxide (IRGACURE 819 (trade name), available from BASF Japan Ltd.), (2,4,6-trimethylbenzoyl)diphenylphosphine oxide (IRGACURE TPO (trade name), available from BASF Japan Ltd.), 2,2-dimethoxy-1,2-diphenylethan-1-one (IRGACURE 651 (trade name), available from BASF Japan Ltd.), 1-[4-(2-hydroxyethoxy)-phenyl]-2-hydroxy-2-methylpropan-1-one, 2-methyl-1-(4-methylthiophenyl)-2-morpholinopropan-1-one, ethylphenyl(2,4,6-trimethylbenzoyl)phosphinate (IRGACURE TPO-L (trade name), available from BASF Japan Ltd.), or ADEKA ARKLS NCI-831E (trade name, available from ADEKA Corporation) can be preferably used as the photoradical polymerization initiator.

The curable composition according to the embodiment of the present invention may contain one type of photoradical polymerization initiator or may contain two or more types of photoradical polymerization initiators.

In a case where the curable composition contains a photoradical polymerization initiator, the content of the photoradical polymerization initiator in the curable composition is preferably 0.01% to 5.0% by mass, more preferably 0.05% to 1.0% by mass, and still more preferably 0.05% to 0.5% by mass.

As long as it does not go against the spirit of the present invention, the curable composition according to the embodiment of the present invention, which contains the compound represented by General Formula (1) or (2), may contain a polymer or a monomer other than the above-mentioned components, a dispersant, a plasticizer, a heat stabilizer, a mold release agent, a solvent, and the like.

In a case where the curable composition is used as a material for imprinting, it is preferable that the content of inorganic particles in the curable composition is kept to 30% by mass or less and it is more preferable that the curable composition does not contain inorganic particles.

From the viewpoint of improving handleability in a case of forming a cured product, in particular improving followability to a mold in a case of carrying out imprinting, and forming a cured product having a higher quality, the viscosity of the curable composition according to the embodiment of the present invention at 60°C is preferably less than 50 mPa-s, more preferably less than 35 mPa·s, still more preferably less than 30 mPa·s, and particularly preferably less than 25 mPa·s. The lower limit value of the viscosity of the curable composition is not particularly limited and is practically 1 mPa·s or more.

The viscosity of the curable composition is a viscosity measured by a method described in Examples which will be described later using a rheometer (for example, trade name: HAAKE RheoStress 6000, manufactured by Thermo Fisher Scientific Inc.). It is noted that 1 mPa·s is 1 cP.

The refractive index of liquid of the curable composition according to the embodiment of the present invention can be evaluated using a refractive index (nD) at 25°C and a wavelength of 589 nm (in the present invention, also simply referred to as "refractive index nD of liquid of the curable composition").

The refractive index nD of liquid of the curable composition according to the embodiment of the present invention can be 1.650 or more and is preferably 1.660 or more and more preferably 1.670 or more. The upper limit value of the refractive index nD of liquid of the curable composition according to the embodiment of the present invention is not particularly limited and is practically 1.750 or less.

The refractive index nD of liquid of the curable composition is a value measured using an Abbe refractometer (for example, trade name: multi-wavelength Abbe refractometer DR-M2 or DR-M4, manufactured by Atago Co., Ltd.), and specifically, a sample for measurement (curable composition) can be prepared and measured according to the description of Examples which will be described later. In addition, Japan Industrial Standards (JIS) B 7090: 1999 Optics and optical instruments-Reference wavelengths (International Organization for Standardization (ISO) 7944: 1998 Optics and optical instruments-Reference wavelengths) can be referred to as appropriate.

The curable composition according to the embodiment of the present invention can be used for manufacturing a cured product that is required to have a high refractive index.

Above all, the curable composition according to the embodiment of the present invention has excellent temporal stability, and therefore can be used as a material for imprinting such as nanoimprinting and can be preferably used for manufacturing a cured product exhibiting a high refractive index.

### [Cured product]

The cured product according to the embodiment of the present invention is a cured product obtained from the curable composition according to the embodiment of the present invention, and is a cured product obtained by curing the curable composition containing the compound represented by General Formula (1) or (2).

The cured product according to the embodiment of the present invention is obtained by carrying out a polymerization reaction of a monomer including the compound represented by General Formula (1) or (2), followed by curing. The cured product according to the embodiment of the present invention may contain unreacted monomers (for example, the compound represented by General Formula (1) or (2), the compound represented by General Formula (7), and the difunctional (meth)acrylic acid thioester compound) and the like.

As described above, the cured product according to the embodiment of the present invention can exhibit a high refractive index.

The refractive index of the cured product according to the embodiment of the present invention can be evaluated using a refractive index (nD) at 25°C and a wavelength of 589 nm (in the present invention, also simply referred to as "refractive index nD of the cured product").

The refractive index nD of the cured product according to the embodiment of the present invention can be 1.670 or more, and is preferably 1.680 or more, more preferably 1.690 or more, and still more preferably 1.700 or more. In the region where the refractive index nD of the cured product is 1.670 or more, a difference in the order of 10⁻³ of the refractive index nD results in a difference in the performance of high refractive index in a case of being applied to an optical member.

The upper limit value of the refractive index nD of the cured product according to the embodiment of the present invention is not particularly limited and is practically 1.800 or less.

The refractive index nD of the cured product is a value measured using an Abbe refractometer (for example, trade name: multi-wavelength Abbe refractometer DR-M2 or DR-M4, manufactured by Atago Co., Ltd.), and specifically, a sample for measurement (cured product) can be produced and measured according to the description of Examples which will be described later. In a case of forming the cured product, a heating step may be employed instead of an ultraviolet irradiating step described in Examples which will be described later, or both of the heating step and the ultraviolet irradiating step may be employed. In addition, JIS B 7090: 1999 Optics and optical instruments-Reference wavelengths (ISO 7944: 1998 Optics and optical instruments-Reference wavelengths) can be referred to as appropriate.

The transmittance of the cured product according to the embodiment of the present invention is preferably 85% or more, more preferably 90% or more, and still more preferably 95% or more over the entire visible light wavelength range of 360 to 830 nm. In addition, the upper limit value of the transmittance of the cured product according to the embodiment of the present invention is not particularly limited and is practically 99% or less.

The transmittance of the cured product is a value of an external transmittance including surface reflection, which is measured using an ultraviolet-visible spectrophotometer (for example, UV-2600 (trade name), manufactured by Shimadzu Corporation) for a cured product having a thickness of 150 µm.

### [Manufacturing method of cured product]

The cured product according to the embodiment of the present invention can be manufactured by a method including a step of photocuring the above-mentioned curable composition. In a case of photocuring, it is preferable that the above-mentioned photoradical polymerization initiator is contained in the curable composition.

With regard to the conditions for photocuring, the description of light irradiation in a diffractive optical element which will be described later can be preferably applied.

In the manufacturing method of the cured product according to the embodiment of the present invention, it is preferable that the curable composition according to the embodiment of the present invention is photocured while being pressed against a mold to obtain a cured product to which a pattern of the mold has been transferred.

From the viewpoint of peelability, a mold that has been subjected to a surface treatment with chromium nitride is preferable as the mold. With regard to the surface treatment of a mold with chromium nitride, for example, the description of the treatment with chromium nitride in paragraph [0108] of WO2019/044863A can be applied as it is, except that "metal mold" is replaced with "mold".

Fig. 1 is a schematic cross-sectional view schematically showing a method of manufacturing the cured product according to the embodiment of the present invention by an imprinting technology using the curable composition according to the embodiment of the present invention. The size, shape, thickness, and the like of a substrate 3, the size, shape, transfer pattern, thickness, and the like of a mold 5, the amount of a curable composition 1 used, and the like can be appropriately adjusted so that a cured product 7 having a desired size, shape, transferred pattern, thickness, and the like is obtained.

As shown in Fig. 1, the cured product 7 can be manufactured in such a manner that (a) first, the curable composition 1 is sandwiched between the mold 5 and the substrate 3, (b) the curable composition 1 is pressed against the mold 5 in a state of being embossed and the curable composition 1 is photocured by ultraviolet irradiation (UV irradiation) to produce the cured product 7 to which a pattern of the mold 5 is transferred, and (c) the obtained cured product 7 and the substrate 3 are released (peeled off) from the mold 5.

The ultraviolet irradiation is not particularly limited as long as the curable composition 1 is cured, and may be carried out from any side of the mold 5 or the substrate 3. It is preferable that the substrate 3 is transparent and the irradiation is carried out from the substrate 3 side.

In addition, the obtained cured product 7 may be used in a form integrated with the substrate 3 or may be used in a form consisting of the cured product 7 after the substrate 3 is peeled off.

With regard to the substrate 3, the description of the transparent substrate in the diffractive optical element which will be described later can be preferably applied.

### [Applications of cured product]

The cured product according to the embodiment of the present invention can be used in a variety of applications, and since the cured product exhibits a high refractive index, it can be preferably used for an optical material, and above all, it can be preferably used for a diffractive optical element. The diffractive optical element according to the embodiment of the present invention can also be used, for example, as a diffractive optical element for a waveguide in augmented reality glasses (AR glasses).

### [Diffractive optical element]

The diffractive optical element according to the embodiment of the present invention is a diffractive optical element which is formed of the cured product according to the embodiment of the present invention and includes a surface having a diffraction grating shape, and is formed by curing the curable composition according to the embodiment of the present invention.

The diffractive optical element according to the embodiment of the present invention preferably has a maximum thickness of 0.05 µm to 100 µm. The maximum thickness is more preferably 0.1 µm to 50 µm and still more preferably 0.2 µm to 30 µm. In addition, a level difference (lattice thickness) of the diffraction grating shape (periodic structure) of the diffractive optical element is preferably 0.05 µm to 100 µm, more preferably 0.05 µm to 50 µm, and still more preferably 0.1 µm to 30 µm. Further, the pitch of the diffraction grating shape of the diffractive optical element may be 0.05 µm to 1 mm, and is also preferably 0.05 µm to 100 µm and preferably varies within the same diffractive optical element depending on the required optical aberration.

The diffractive optical element can be manufactured, for example, by the following procedure.

The curable composition is sandwiched between a transparent substrate and a surface processed into a diffraction grating shape of a mold such as a metal mold, which has the surface processed into a diffraction grating shape. Thereafter, the curable composition may be pressurized and stretched to a desired extent. In the sandwiched state, the curable composition is cured by irradiation with light from the transparent substrate side. The cured product is then released from the mold such as a metal mold. After the release from the mold, the cured product may be further irradiated with ultraviolet rays from a side opposite to the transparent substrate side.

Examples of the transparent substrate include flat glass such as BK glass, and a flat transparent resin (a (meth)acrylic resin, a polycarbonate resin, polyethylene terephthalate, or the like). The surface of the transparent substrate may be subjected to a surface treatment such as an ozone treatment.

The transparent substrate used in the above-mentioned manufacturing may be included in the diffractive optical element as it is, or may be peeled off.

The light used for the light irradiation to cure the curable composition is preferably ultraviolet rays or visible rays and more preferably ultraviolet rays. For example, a metal halide lamp, a low pressure mercury lamp, a high pressure mercury lamp, an ultra-high pressure mercury lamp, a germicidal lamp, a xenon lamp, or a light emitting diode (LED) light source lamp is suitably used. The illuminance of ultraviolet light used for the light irradiation to cure the curable composition is preferably 1 to 100 mW/cm², more preferably 1 to 75 mW/cm², and still more preferably 5 to 50 mW/cm². The curable composition may be irradiated multiple times with ultraviolet light having different illuminance. The exposure amount of ultraviolet light is preferably 0.4 to 10 J/cm², more preferably 0.5 to 5 J/cm², and still more preferably 1 to 3 J/cm². The atmosphere during the light irradiation is preferably air or an inert gas-purged atmosphere, and more preferably an atmosphere in which air has been purged with nitrogen until the oxygen concentration reaches 1% or less.

Using the curable composition according to the embodiment of the present invention as a material for imprinting, the diffraction grating shape can be produced in such a manner that the material is pressed against a mold having a desired pattern with a size of nanometers to several hundred micrometers in a state of being embossed, followed by photocuring to produce the cured product according to the embodiment of the present invention to which the pattern of the mold has been transferred, and then the cured product is released from the mold.

With regard to the conditions for the photo curing, the description in the above-mentioned manufacturing method of a cured product and the description in the above-mentioned manufacture of a diffractive optical element can be applied.

For the points other than the above-mentioned points, any commonly used description of imprinting can be adopted without any particular limitation, and for example, a nanoimprint technology handbook (edited by the Japan Society of Applied Physics and Nanoimprint Technology Research Association, published by Ohmsha, Ltd., December 1, 2019) can be referred to.

### Examples

Hereinafter, the present invention will be described in more detail with reference to Examples. The materials, amounts used, proportions, treatment details, treatment procedures, and the like shown in the Examples below can be modified as appropriate without departing from the spirit of the present invention. Therefore, the scope of the present invention should not be construed as being limited to the specific examples shown below. In the following description, room temperature means 25°C unless otherwise specified.

All steps from the preparation of the curable composition to the production or evaluation test of the cured product were carried out in an environment where a yellow lamp was used as lighting.

### [Synthesis Example]

The compound represented by General Formula (1) or (2) and the compound represented by General Formula (7) were synthesized as follows.

### [Synthesis Example 1: Synthesis of compound (A-4) and compound (A-5)]

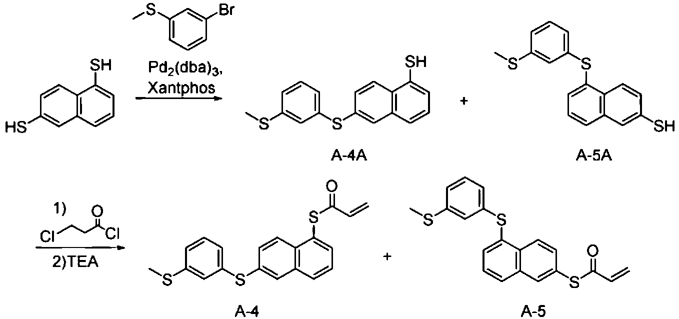

### <Synthesis of compound (A-4A) and compound (A-5A)>

While mixing 12.8 g (66.8 mmol) of 1,6-naphthalenedithiol, 12.9 g (63.6 mmol) of 3-bromothioanisole, 95 mL of N,N-dimethylacetamide (DMAc), and 18.0 g (130.4 mmol) of potassium carbonate, nitrogen purging was carried out, and then 2.91 g (3.18 mmol) of tris(dibenzylideneacetone) dipalladium (Pd₂(dba)₃) and 3.68 g (6.36 mmol) of 4,5-bis(diphenylphosphino)-9,9-dimethylxanthene (Xantphos) were added thereto, and the mixture was heated such that an internal temperature (liquid temperature) was 120°C. After stirring for 5 hours, 800 mL of ethyl acetate and 800 mL of 1 N hydrochloric acid were added thereto, and the insoluble matter was filtered off, followed by washing and liquid separation. Next, 800 mL of water was added thereto, followed by stirring and then washing and liquid separation. Dehydration with magnesium sulfate, filtration, and concentration were carried out to obtain an oily composition which was then purified by column chromatography to obtain 5.2 g of a mixture of a compound (A-4A) and a compound (A-5A). The yield was 26%.

### <Synthesis of compound (A-4) and compound (A-5)>

3.0 g (9.5 mmol) of a mixture of the compound (A-4A) and the compound (A-5A) and 5 mL of N,N-dimethylacetamide (DMAc) were cooled while being mixed such that the internal temperature (liquid temperature) was 0°C. 1.33 g (10.5 mmol) of 3-chloropropionyl chloride (3CPC) was added dropwise thereto such that the liquid temperature did not exceed 7°C, and the mixture was heated such that the internal temperature (liquid temperature) was 25°C. After stirring for 1 hour, the mixture was cooled to 0°C, 2.31 g (22.9 mmol) of triethylamine (TEA) was added dropwise thereto such that the liquid temperature did not exceed 7°C, and the mixture was heated such that the internal temperature (liquid temperature) was 25°C. After stirring for 1 hour, 20 mL of ethyl acetate and 20 mL of 1 N hydrochloric acid were added thereto, and the insoluble matter was filtered off, followed by washing and liquid separation. Next, 20 mL of a 5% sodium hydrogen carbonate aqueous solution was added thereto, followed by stirring and then washing and liquid separation. Dehydration with magnesium sulfate, filtration, and concentration were carried out to obtain an oily composition which was then purified by column chromatography to obtain 1.83 g of a mixture of a compound (A-4) and a compound (A-5). The yield was 52%. The concentrations of each of the compound (A-4) and the compound (A-5) alone were calculated from the areas of the compound (A-4) and the compound (A-5) at a wavelength of 254 nm by analysis with high performance liquid chromatography (HPLC), and the compositional ratio calculated and obtained from these results was compound (A-4):compound (A-5) = 45% by mass:55% by mass.
Compound (A-4) ¹H-NMR (300MHz, CDCl₃): δ (ppm) 2.45 (s, 3H), 5.8-5.9 (m, 1H), 6.4-6.6 (m, 2H), 7.1-7.5 (m, 8H), 7.9-8.1 (m, 2H)
Compound (A-5) ¹H-NMR (300MHz, CDCl₃): δ (ppm) 2.45 (s, 3H), 5.8-5.9 (m, 1H), 6.4-6.6 (m, 2H), 7.1-7.5 (m, 8H), 7.9-8.1 (m, 1H), 8.2-8.3 (m, 1H)

### [Synthesis Example 2: Synthesis of compound (A-21) and compound (A-22)]

A mixture of a compound (A-21) and a compound (A-22) which will be described later was synthesized in the same manner as in Synthesis Example 1, except that 3-bromothioanisole was changed to bromobenzene (yield: 28%). The concentrations of each of the compound (A-21) and the compound (A-22) alone were calculated from the areas of the compound (A-21) and the compound (A-22) at a wavelength of 254 nm by analysis with high performance liquid chromatography (HPLC), and the compositional ratio calculated and obtained from these results was compound (A-21):compound (A-22) = 45% by mass:55% by mass.
Compound (A-21) ¹H-NMR (300MHz, CDCl₃): δ (ppm) 5.8-5.9 (m, 1H), 6.4-6.6 (m, 2H), 7.1-7.7 (m, 9H), 7.9-8.1 (m, 2H)
Compound (A-22) ¹H-NMR (300MHz, CDCl₃): δ (ppm) 5.8-5.9 (m, 1H), 6.4-6.6 (m, 2H), 7.1-7.7 (m, 9H), 7.9-8.1 (m, 1H), 8.2-8.3 (m, 1H)

### [Synthesis Example 3: Synthesis of compound (B-4) and compound (B-5)]

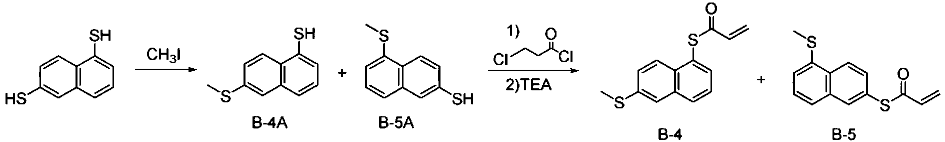

### <Synthesis of compound (B-4A) and compound (B-5A)>

18.6 g (96.9 mmol) of 1,6-naphthalenedithiol, 13.7 g (96.9 mmol) of methyl iodide, and 240 mL of N,N-dimethylacetamide (DMAc) were cooled while being mixed such that the internal temperature (liquid temperature) was 0°C. 13.2 g (101.8 mmol) of N,N-diisopropylethylamine (DIPEA) was added dropwise thereto such that the liquid temperature did not exceed 7°C, and then the mixture was heated such that the internal temperature (liquid temperature) was 25°C. After stirring for 1 hour, 800 mL of ethyl acetate and 800 mL of 1 N hydrochloric acid were added thereto, followed by washing and liquid separation. Next, 800 mL of water was added thereto, followed by stirring and then washing and liquid separation. Dehydration with magnesium sulfate, filtration, and concentration were carried out to obtain an oily composition which was then purified by column chromatography to obtain 6.8 g of a mixture of a compound (B-4A) and a compound (B-5A). The yield was 34%.

### <Synthesis of compound (B-4) and compound (B-5)>

5.0 g (24.2 mmol) of a mixture of the compound (B-4A) and the compound (B-5A) and 12 mL of N,N-dimethylacetamide (DMAc) were cooled while being mixed such that the internal temperature (liquid temperature) was 0°C. 3.38 g (26.7 mmol) of 3-chloropropionyl chloride (3CPC) was added dropwise thereto such that the liquid temperature did not exceed 7°C, and the mixture was heated such that the internal temperature (liquid temperature) was 25°C. After stirring for 1 hour, the mixture was cooled to 0°C, 5.89 g (58.1 mmol) of triethylamine (TEA) was added dropwise thereto such that the liquid temperature did not exceed 7°C, and the mixture was heated such that the internal temperature (liquid temperature) was 25°C. After stirring for 1 hour, 40 mL of ethyl acetate and 40 mL of 1 N hydrochloric acid were added thereto, and the insoluble matter was filtered off, followed by washing and liquid separation. Next, 40 mL of a 5% sodium hydrogen carbonate aqueous solution was added thereto, followed by stirring and then washing and liquid separation. Dehydration with magnesium sulfate, filtration, and concentration were carried out to obtain an oily composition which was then purified by column chromatography to obtain 3.2 g of a mixture of a compound (B-4) and a compound (B-5). The yield was 51%. The concentrations of each of the compound (B-4) and the compound (B-5) alone were calculated from the areas of the compound (B-4) and the compound (B-5) at a wavelength of 254 nm by analysis with high performance liquid chromatography (HPLC), and the compositional ratio calculated and obtained from these results was compound (B-4):compound (B-5) = 43% by mass:57% by mass. In addition, a part of this mixture was purified again by column chromatography (eluent: a mixed liquid of toluene and hexane) to isolate the compound (B-4) and the compound (B-5).
Compound (B-4) ¹H-NMR (300MHz, CDCl₃): δ (ppm) 2.58 (s, 3H), 5.8-5.9 (m, 1H), 6.4-6.6 (m, 2H), 7.3-7.5 (m, 2H), 7.6-7.7 (m, 2H), 7.8-7.9 (m, 1H), 8.0-8.1 (m, 1H)
Compound (B-5) ¹H-NMR (300MHz, CDCl₃): δ (ppm) 2.58 (s, 3H), 5.8-5.9 (m, 1H), 6.4-6.6 (m, 2H), 7.4-7.5 (m, 3H), 7.6-7.7 (m, 1H), 7.9-8.0 (m, 1H), 8.34 (d, 1H)

### [Synthesis Example 4: Synthesis of mixture of compound (B-4), compound (B-5), compound (C-1), and compound (C-2)]

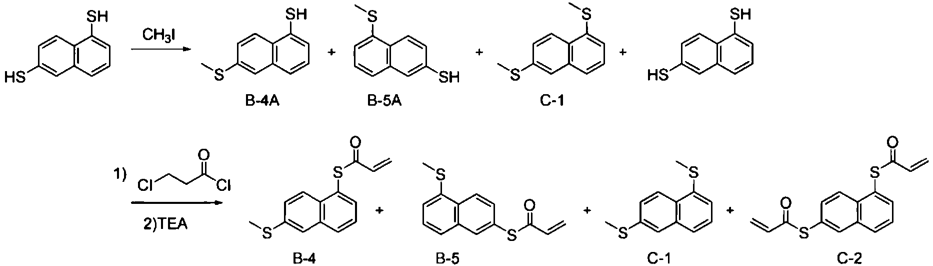

### <Synthesis of mixture of compound (B-4A), compound (B-5A), compound (C-1), and 1,6-naphthalenedithiol>

18.6 g (96.9 mmol) of 1,6-naphthalenedithiol, 13.7 g (96.9 mmol) of methyl iodide, and 240 mL of N,N-dimethylacetamide (DMAc) were cooled while being mixed such that the internal temperature (liquid temperature) was 0°C. 13.2 g (101.8 mmol) of N,N-diisopropylethylamine (DIPEA) was added dropwise thereto such that the liquid temperature did not exceed 7°C, and then the mixture was heated such that the internal temperature (liquid temperature) was 25°C. After stirring for 1 hour, 800 mL of ethyl acetate and 800 mL of 1 N hydrochloric acid were added thereto, followed by washing and liquid separation. Next, 800 mL of water was added thereto, followed by stirring and then washing and liquid separation. Dehydration with magnesium sulfate, filtration, and concentration were carried out to obtain 19.5 g of a mixture of a compound (B-4A), a compound (B-5A), a compound (C-1), and 1,6-naphthalenedithiol as an oily composition. The yield was 98%.

### <Synthesis of mixture of compound (B-4), compound (B-5), compound (C-1), and compound (C-2)>

5.0 g (24.2 mmol) of a mixture of the compound (B-4A), the compound (B-5A), the compound (C-1), and 1,6-naphthalenedithiol and 12 mL of N,N-dimethylacetamide (DMAc) were cooled while being mixed such that the internal temperature (liquid temperature) was 0°C. 3.38 g (26.7 mmol) of 3-chloropropionyl chloride (3CPC) was added dropwise thereto such that the liquid temperature did not exceed 7°C, and the mixture was heated such that the internal temperature (liquid temperature) was 25°C. After stirring for 1 hour, the mixture was cooled to 0°C, 5.89 g (58.1 mmol) of triethylamine (TEA) was added dropwise thereto such that the liquid temperature did not exceed 7°C, and the mixture was heated such that the internal temperature (liquid temperature) was 25°C. After stirring for 1 hour, 40 mL of ethyl acetate and 40 mL of 1 N hydrochloric acid were added thereto, and the insoluble matter was filtered off, followed by washing and liquid separation. Next, 40 mL of a 5% sodium hydrogen carbonate aqueous solution was added thereto, followed by stirring and then washing and liquid separation. Dehydration with magnesium sulfate, filtration, and concentration were carried out to obtain an oily composition which was then purified by column chromatography to obtain 5.4 g of a mixture of a compound (B-4), a compound (B-5), a compound (C-1), and a compound (C-2). The yield was 85%. The concentrations of each of the compound (B-4), the compound (B-5), the compound (C-1), and the compound (C-2) alone were calculated from the areas of the compound (B-4), the compound (B-5), the compound (C-1), and the compound (C-2) at a wavelength of 254 nm by analysis with high performance liquid chromatography (HPLC), and the compositional ratio calculated and obtained from these results was compound (B-4):compound (B-5):compound (C-1):compound (C-2) = 20% by mass:25% by mass:25% by mass:30% by mass.

### [Synthesis Example 5: Synthesis of mixture of compound (B-4), compound (B-5), and compound (C-2)]

In the purification by column chromatography in Synthesis Example 4, the compound (C-1) was excluded, and the compound (B-4), the compound (B-5), and the compound (C-2) were isolated (yield: 63%). The concentrations of each of the compound (B-4), the compound (B-5), and the compound (C-2) alone were calculated from the areas of the compound (B-4), the compound (B-5), and the compound (C-2) at a wavelength of 254 nm by analysis with high performance liquid chromatography (HPLC), and the compositional ratio calculated and obtained from these results was compound (B-4):compound (B-5):compound (C-2) = 28% by mass:34% by mass:38% by mass.

### [Synthesis Example 6: Synthesis of compound (C-1)]

5.0 g (26.0 mmol) of 1,6-naphthalenedithiol, 7.4 g (52.0 mmol) of methyl iodide, and 65 mL of N,N-dimethylacetamide (DMAc) were cooled while being mixed such that the internal temperature (liquid temperature) was 0°C. 7.1 g (54.6 mmol) of N,N-diisopropylethylamine (DIPEA) was added dropwise thereto such that the liquid temperature did not exceed 7°C, and then the mixture was heated such that the internal temperature (liquid temperature) was 25°C. After stirring for 1 hour, 50 mL of ethyl acetate and 50 mL of 1 N hydrochloric acid were added thereto, followed by washing and liquid separation. Next, 50 mL of water was added thereto, followed by stirring and then washing and liquid separation. Dehydration with magnesium sulfate, filtration, and concentration were carried out, followed by purification with column chromatography to obtain 5.4 g of a compound (C-1). The yield was 95%.
¹H-NMR (300MHz, CDCl₃): δ (ppm) 2.56 (s, 3H), 2.59 (s, 3H), 7.3-7.4 (m, 1H), 7.4-7.5 (m, 2H), 7.5-7.6 (m, 2H), 8.2 (d, 1H)

### [Synthesis Example 7: Synthesis of compound (C-2)]

5.0 g (26.0 mmol) of 1,6-naphthalenedithiol and 12.5 mL of N,N-dimethylacetamide (DMAc) were cooled while being mixed such that the internal temperature (liquid temperature) was 0°C. 7.3 g (57.2 mmol) of 3-chloropropionyl chloride (3CPC) was added dropwise thereto such that the liquid temperature did not exceed 7°C, and the mixture was heated such that the internal temperature (liquid temperature) was 25°C. After stirring for 8 hours, the mixture was cooled to 0°C, 11.6 g (114 mmol) of triethylamine (TEA) was added dropwise thereto such that the liquid temperature did not exceed 7°C, and the mixture was heated such that the internal temperature (liquid temperature) was 25°C. After stirring for 1 hour, 50 mL of ethyl acetate and 50 mL of 1 N hydrochloric acid were added thereto, and the insoluble matter was filtered off, followed by washing and liquid separation. Next, 50 mL of a 5% sodium hydrogen carbonate aqueous solution was added thereto, followed by stirring and then washing and liquid separation. Dehydration with magnesium sulfate, filtration, and concentration were carried out to obtain an oily composition which was then purified by column chromatography to obtain 6.6 g of a compound (C-2). The yield was 85%.
¹H-NMR (300MHz, CDCl₃): δ (ppm) 5.8-5.9 (m, 2H), 6.4-6.6 (m, 4H), 7.5-7.6 (m, 2H), 7.7-7.8 (m, 1H), 7.9-8.0 (m, 1H), 8.05 (s, 1H), 8.23 (d, 1H)

### [Example 1: Preparation of curable composition]

The compound represented by General Formula (1) or (2), the compound represented by General Formula (7), or the comparative compound, and a difunctional (meth)acrylic acid thioester compound M as a diluent monomer were mixed so as to have the composition shown in Table 1 which will be given later. The mixture was dissolved in ethyl acetate, followed by concentration at 60°C under a reduced pressure of 40 hPa until the ethyl acetate disappeared. A photoradical polymerization initiator (referred to as "Photopolymerization initiator" in Table 1) was added to the obtained concentrate, and the mixture was stirred to be homogeneous while being heated to 60°C, thereby preparing a curable composition.

The curable composition Nos. 101 to 107 are compositions containing the compound represented by General Formula (1) or (2) according to the embodiment of the present invention, and the curable composition No. c11 is a comparative composition containing a comparative compound instead of the compound represented by General Formula (1) or (2) according to the embodiment of the present invention.

### [Example 2: Production of cured product]

The above-prepared curable composition was sandwiched between glass plates subjected to a hydrophobization treatment, such that a film thickness of a cured product was 150 µm, irradiated with ultraviolet (UV) rays using a UV irradiation device (EXECURE 3000 (trade name), manufactured by HOYA CANDEO OPTRONICS CORPORATION) under conditions of an integrated light amount of 1.2 J/cm² and an illuminance of 5 mW/cm² in an atmosphere purged with nitrogen (N₂) so that the oxygen concentration was 1% or less, and then peeled off from the glass plates to produce a cured product.

The values of the transmittance of the cured products having a film thickness of 150 µm of the above-produced curable composition Nos. 101 to 107, measured by the above-mentioned measuring method, were all 85% or more over the entire visible light wavelength range of 360 to 830 nm.

### [Evaluation 1] Refractive index

The refractive index (nD) of a sample for measurement at a wavelength of 589 nm was measured under a condition of 25°C using a multi-wavelength Abbe refractometer DR-M2 or DR-M4 (trade name, manufactured by Atago Co., Ltd.). As the sample for measurement, the above-prepared curable composition was used for the refractive index nD of liquid of the composition, and the above-prepared cured product having a film thickness of 150 µm was used for the refractive index nD of the cured product. The refractive index nD of the cured product was evaluated according to the following standards.

The results are summarized in Table 1. With regard to the refractive index nD of the cured product, specific values are shown in parentheses in the table together with the evaluation results.

### - Evaluation standards for refractive index nD of cured product -

A: 1.700 ≤ nD
B: 1.690 ≤ nD< 1.700
C: 1.680 ≤ nD< 1.690
D: 1.670 ≤ nD< 1.680
E: nD < 1.670

### [Evaluation 2] Viscosity measurement

The viscosity η of the above-prepared curable composition at a shear rate of 10 s⁻¹ and 60°C was measured using a rheometer (trade name: HAAKE RheoStress 6000, manufactured by Thermo Fisher Scientific Inc.), and evaluated according to the following standards. 1 cP is 1 mPa·s.

The results are summarized in Table 1. Specific values are shown in parentheses together with the evaluation results. In this regard, the viscosity values in the table are listed without the unit "cP".

### - Evaluation standards for viscosity -

A: η < 25 cP
B: 25 cP ≤ η < 30 cP
C: 30 cP ≤ η < 35 cP
D: 35 cP ≤ η < 50 cP

### [Evaluation 3] Temporal stability of liquid

The above-prepared curable composition was stored at 25°C for one week and whether or not any change in crystal precipitation and/or turbidity occurred over time was visually observed, and the temporal stability of the liquid was evaluated according to the following standards. The storage was carried out in an environment in which a yellow lamp was used as lighting.

### - Evaluation standards for temporal stability of liquid -

A: There was no change in either crystal precipitation or turbidity, and the liquid remained transparent.
B: There was a slight change in at least one of turbidity or crystal precipitation.
C: There was an occurrence of at least one of turbidity or crystal precipitation, and the change was obvious.

**[Table 1]**

| Curable composition No. | | 101 | 102 | 103 | 104 | 105 | 106 | 107 | c11 |
|---|---|---|---|---|---|---|---|---|---|
| Main monomer | Type | A-4/A-5 | B-4/B-5 | A-21/A-22 | B-4 | B-5 | B-4/B-5/C-2 | B-4/B-5/C-1/C-2 | Z-1 |
| | Formulation amount | 79.8 wt% | 79.8 wt% | 79.8 wt% | 79.8 wt% | 79.8 wt% | 79.8 wt% | 79.8 wt% | 79.8 wt% |
| | Ratio of each component | 45 wt%/55 wt% | 43 wt%/57 wt% | 45 wt%/55 wt% | 100 wt% | 100 wt% | 28 wt%/34 wt%/38 wt% | 20 wt%/25 wt%/25 wt%/30 wt% | 100 wt% |
| Diluent monomer | Type | M-1 | M-1 | M-1 | M-1 | M-1 | M-1 | M-1 | M-1 |
| | Formulation amount | 19.9 wt% | 19.9 wt% | 19.9 wt% | 19.9 wt% | 19.9 wt% | 19.9 wt% | 19.9 wt% | 19.9 wt% |
| Photopolymerization initiator IrgTPO | Formulation amount | 0.3 wt% | 0.3 wt% | 0.3 wt% | 0.3 wt% | 0.3 wt% | 0.3 wt% | 0.3 wt% | 0.3 wt% |
| Refractive index nD of liquid of composition | | 1.705 | 1.671 | 1.694 | 1.671 | 1.671 | 1.672 | 1.672 | 1.634 |
| Refractive index nD of cured product | | A (1.735) | A (1.704) | A (1.724) | A (1.704) | A (1.704) | A (1.705) | A (1.701) | E (1.656) |
| Viscosity at 60°C | | A (24) | A(18) | A (24) | A (20) | A(18) | A (17) | A(17) | C (31) |
| Temporal stability of liquid | | A | A | A | A | B | A | A | C |

### <Notes for table>

The formulation amount wt% of each component described in the column of each component means % by mass.

In addition, the ratio of each component described in the column of the main monomer means a content ratio of each compound constituting the main monomer, and wt% means % by mass. In a case where the main monomer is a mixture of two or more types of compounds, the content ratio of each compound is described in the order of the compounds described in the column of the type.

The description of the main monomer in the curable composition Nos. 101 to 103 means each of mixtures of the compounds represented by General Formula (1) or (2), which are obtained in Synthesis Examples 1 to 3 described above, and the formulation amount means a formulation amount as a mixture.

In addition, the description of the main monomer in the curable composition Nos. 106 and 107 means each of mixtures of the compound represented by General Formula (1) or (2) and the compound represented by General Formula (7), which are obtained in Synthesis Examples 4 and 5 described above, and the formulation amount means a formulation amount as a mixture.

### The components in the table are as follows.

Main monomer: a compound represented by General Formula (1) or (2), a compound represented by General Formula (7), or a comparative compound, which are shown below.

### (Compound represented by General Formula (1) or (2))

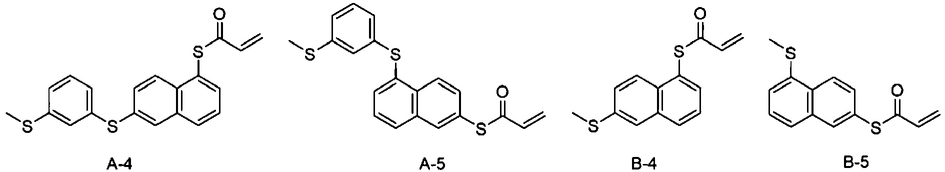

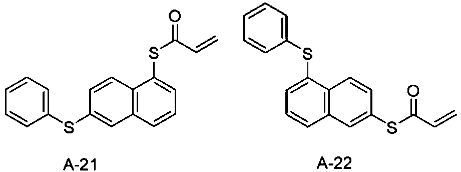

### (Compound represented by General Formula (7))

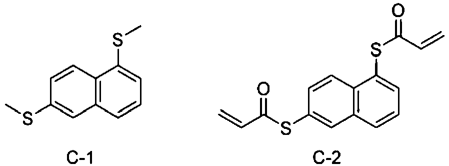

### (Comparative compound)

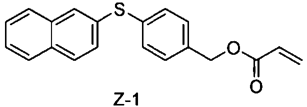

Comparative compound Z-1 is the polymerizable compound (A-1) described in paragraph [0129] of JP2018-104696A, which is used in Examples thereof.

Diluent monomer: a difunctional (meth)acrylic acid thioester compound M shown below.

### (Difunctional (meth)acrylic acid thioester compound M)

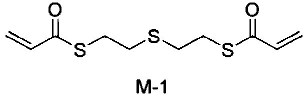

### (Photopolymerization initiator)

IrgTPO: IRGACURE TPO (trade name, manufactured by BASF Japan Ltd., available as Omnirad TPO H (trade name, manufactured by IGM Resins B.V.)), diphenyl(2,4,6-trimethylbenzoyl)phosphine oxide

From the results in Table 1, the following was found.

The comparative compound Z-1 is not the compound represented by General Formula (1) or (2) according to the embodiment of the present invention in that it has an acryloyloxy group as a functional group, the acryloyloxy group and a naphthalene ring are linked by -S-phenylene-CH₂-, and the naphthalene ring does not have a specific substituent represented by Q¹ or Q². The curable composition No. c11 containing the comparative compound Z-1 was inferior in temporal stability of the composition (liquid), and the refractive index nD of the obtained cured product was also low and not at a sufficient level, which was inferior.

On the other hand, the curable composition Nos. 101 to 107 containing the compound represented by General Formula (1) or (2) according to the embodiment of the present invention were excellent in temporal stability of the composition (liquid), and the obtained cured product had a high refractive index nD of 1.701 or more, which was excellent. In addition, since the curable composition No. 105 contained the compound (B-5) which is a crystalline compound as shown in Table 2 which will be given later, slight crystal precipitation was observed over time, but the curable composition No. 102 containing the compound (B-5) in combination with (B-4) did not show any change in either crystal precipitation or turbidity over time, indicating more excellent temporal stability of the composition (liquid). In addition, the curable composition Nos. 106 and 107, in which a mixture of three or more types of compounds was used as the main monomer and combined with a diluent monomer, also had a high refractive index nD and exhibited more excellent temporal stability of the composition (liquid).

### [Example 3: Evaluation of compounds]

The following evaluations of crystal precipitation properties and coloration were carried out for sample Nos. 201 to 211, r21, and r22 of the compounds synthesized above as main monomers or mixtures thereof. The results are summarized in Tables 2-1 and 2-2 (hereinafter, referred to as "Table 2").

### [Evaluation 4] Crystal precipitation properties

Approximately 200 mg of a sample was placed in a 1.5 mL vial, heated in a state where the sample temperature was 70°C to be completely melted, and then cooled at a rate of 10 °C/min such that the sample temperature was 25°C. Thereafter, changes over time were observed while maintaining the sample temperature of 25°C, the time until the entire melted liquid was crystallized was measured, and the crystal precipitation properties were evaluated according to the following evaluation standards.

### - Evaluation standards for crystal precipitation properties -

A: No crystal precipitation was observed even after 96 hours.
B: Crystals were precipitated in the entire area in a period of time of more than 48 hours and up to 96 hours.
C: Crystals were precipitated in the entire area in a period of time of more than 24 hours and up to 48 hours.
D: Crystals were precipitated in the entire area in a period of time of more than 12 hours and up to 24 hours.
E: Crystals were precipitated in the entire area in a period of time of up to 12 hours.

### [Evaluation 5] Coloration

Approximately 30 mg of a sample was placed on a slide glass, heated to a sample temperature of 70°C to be completely melted, another slide glass was superimposed thereon to sandwich the sample, and the sample was pressed and spread to a thickness of 150 µm. Thereafter, using an ultraviolet-visible spectrophotometer (trade name: UV2600, manufactured by Shimadzu Corporation), the transmittance (T%) at a wavelength of 450 nm was measured using one piece of the above-mentioned slide glass as a reference, and the coloration was evaluated according to the following evaluation standards.

### - Evaluation standards for coloration -

A: T% ≥ 96%
B: 96% > T% ≥ 95%
C: 95% > T% ≥ 93%
D: 93% > T% ≥ 91%
E: T% < 91%

### [Table 2]

**Table 2-1**

| Sample No. | | 201 | 202 | r21 | r22 | 203 | 204 | 205 | 206 | 207 |
|---|---|---|---|---|---|---|---|---|---|---|
| Compound | Type | B-5 | B-4 | C-1 | C-2 | B-5/C-1 | B-5/C-2 | B-4/B-5 | B-4/C-1 | B-4/C-2 |
| | Formulation amount | 100 wt% | 100 wt% | 100 wt% | 100 wt% | 50 wt%/50 wt% | 50 wt%/50 wt% | 50 wt%/50 wt% | 50 wt%/50 wt% | 50 wt%/50 wt% |
| Coloration | | A | D | A | A | A | A | B | B | B |
| Crystal precipitation properties | | E | A | E | E | C | C | B | B | B |

**Table 2-2**

| Sample No. | | 208 | 209 | 210 | 211 |
|---|---|---|---|---|---|
| Compound | Type | B-5/C-1/C-2 | B-4/B-5/C-2 | B-4/B-5/C-2 | B-4/B-5/C-1/C-2 |
| | Formulation amount | 100/3 wt% formulated for each compound | 100/3 wt% formulated for each compound | 28 wt%/34 wt%/38 wt% | 20 wt%/25 wt%/25 wt%/30 wt% |
| Coloration | | A | A | A | A |
| Crystal precipitation properties | | A | A | A | A |

### <Notes for table>

Each compound in the table is as described for each main monomer in the notes of Table 1.

In addition, the formulation amount described in the column of the compound means a formulation ratio of each compound constituting a sample, and wt% means % by mass. In a case where the sample is a mixture of two or more types of compounds, the formulation ratio of each compound is described in the order of the compounds described in the column of the type.

From the results in Table 2, the following was found.

In a case where the compound was used alone, the compound (B-5), the compound (C-1), and the compound (C-2) had a transmittance of 96% or more at a wavelength of 450 nm and exhibited almost no coloration, but were crystalline compounds, and the compound (B-4) was a liquid, but had absorption on a long wavelength side and therefore had a transmittance of 91% or more and less than 93% at a wavelength of 450 nm and exhibited slight coloration (see sample Nos. 201, 202, r21, and r22).

On the other hand, in a case where a mixture of two types of compounds was used, crystal precipitation could be suppressed and temporal stability was improved as compared with a case where the compound was used alone (see sample Nos. 203 and 204 with respect to sample Nos. 201, r21, and r22, and sample Nos. 205 to 207 with respect to sample Nos. 202, r21, and r22). In particular, the sample Nos. 205 to 207, which are mixtures of two types of compounds containing the compound (B-4), had a higher level of temporal stability and were more excellent than the sample Nos. 203 and 204, which are mixtures of two types of compounds not containing the compound (B-4).

Furthermore, as a result of examining mixtures of three or more types of compounds, it was found that crystal precipitation was further suppressed as compared with the mixtures of two types of compounds (see sample Nos. 208 to 211 with respect to sample Nos. 203 to 207). In addition, by using a mixture of three or more types of compounds, the formulation amount of the compound (B-4) could be reduced, and coloration could also be suppressed.

Each sample shown in Table 2 and IRGACURE TPO (trade name, manufactured by BASF Japan Ltd., available as Omnirad TPO H (trade name, manufactured by IGM Resins B.V.)) as a photoradical polymerization initiator were formulated at a ratio of 99.7% by mass:0.3% by mass, mixed at 60°C, and then cooled to 25°C to obtain a curable composition. In a case where the curable compositions thus obtained were evaluated for the crystal precipitation properties by the method described in Evaluation 4, the amount of crystal precipitation was suppressed to the same level or a lower level than that of each sample shown in Table 2.

Among these curable compositions, approximately 30 mg of a curable composition obtained using each of sample Nos. 202 to 211, which were evaluated as C or higher in terms of crystal precipitation properties and in which crystal precipitation was suppressed, was placed on a slide glass, another slide glass was superimposed thereon to sandwich the sample, the sample was pressed and spread to a thickness of 150 µm, and then UV curing was carried out under the conditions described in Example 2 described above. It was confirmed that this resulted in a cured film that can be applied to an optical member.

That is, as a result of measuring the refractive index (nD) at a wavelength of 589 nm for the produced cured film (cured product) by the method described in Evaluation 1 of Example 2, the refractive index nD of the cured product was 1.71 or more in a case where any of sample Nos. 202 to 211 was used, and a cured film that could be applied to an optical member was obtained.

In addition, the same procedure was also carried out on a level where the type of the photoradical polymerization initiator was changed. Each sample shown in Table 2 and the photoradical polymerization initiator were formulated at a ratio of 99.0% to 99.8% by mass:0.2% to 1.0% by mass so that the total amount thereof was 100% by mass, mixed at 60°C, and then cooled to 25°C to obtain a curable composition.

In addition, three forms were carried out in a case where IRGACURE TPO-L (trade name, manufactured by BASF Japan Ltd., available as Omnirad TPO-L (trade name, manufactured by IGM Resins B.V.)) was used as the photoradical polymerization initiator, in a case where IRGACURE 819 (trade name, manufactured by BASF Japan Ltd., available as Omnirad 819 (trade name, manufactured by IGM Resins B.V.)) was used as the photoradical polymerization initiator, and in a case where ADEKA ARKLS NCI-831E (trade name, manufactured by ADEKA Corporation) was used as the photoradical polymerization initiator.

In a case where the curable compositions thus obtained were evaluated for the crystal precipitation properties by the method described in Evaluation 4, the amount of crystal precipitation was suppressed to the same level or a lower level than that of each sample shown in Table 2.

Among these curable compositions, approximately 30 mg of a curable composition obtained using each of sample Nos. 202 to 211 in which crystal precipitation was suppressed was placed on a slide glass, another slide glass was superimposed thereon to sandwich the sample, the sample was pressed and spread to a thickness of 150 µm, and then UV curing was carried out under the conditions described in Example 2 described above. It was confirmed that a cured film that can be applied to an optical member could be obtained. That is, as a result of measuring the refractive index (nD) at a wavelength of 589 nm for the produced cured film (cured product) by the method described in Evaluation 1 of Example 2, the refractive index nD of the cured product was 1.71 or more in all three forms in which the type of the photoradical polymerization initiator was changed, even in a case where any of sample Nos. 202 to 211 was used, and a cured film that could be applied to an optical member was obtained.

As some specific examples of the above-mentioned examples in which the type of the photoradical polymerization initiator was changed, in an example in which sample No. 210 shown in Table 2 and IRGACURE 819 (trade name, manufactured by BASF Japan Ltd., available as Omnirad 819 (trade name, manufactured by IGM Resins B.V.)) were formulated at a ratio of 99.7% by mass:0.3% by mass, the refractive index (nD) of the cured product at a wavelength of 589 nm, which was measured by the method described in Evaluation 1 of Example 2, was 1.717. In addition, in an example in which Sample No. 211 shown in Table 2 and IRGACURE 819 (trade name, manufactured by BASF Japan Ltd., available as Omnirad 819 (trade name, manufactured by IGM Resins B.V.)) were formulated at a ratio of 99.7% by mass:0.3% by mass, the refractive index (nD) of the cured product at a wavelength of 589 nm, which was measured by the method described in Evaluation 1 of Example 2, was 1.713.

Although the present invention has been described in conjunction with embodiments thereof, it is not intended to limit the present invention to any of the details of the above description unless specifically stated otherwise, but it is believed that the present invention should be construed broadly without departing from the spirit and scope of the invention as set forth in the appended "WHAT IS CLAIMED IS".

The present application claims priority on the basis of JP2023-012624 filed in Japan on January 31, 2023, JP2023-123517 filed in Japan on July 28, 2023, and JP2024-005032 filed in Japan on January 17, 2024, the contents of which are incorporated herein by reference in their entirety as a part of the present specification.

### Explanation of References

1: curable composition
3: substrate
5: mold
7: cured product

## Claims

1. A curable composition comprising:
a compound represented by General Formula (1) or (2), 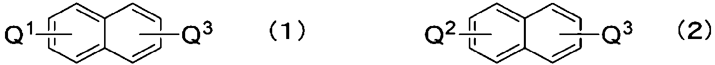
in the formulae, Q¹ represents a group represented by Formula (qla) or (qlb), Q² represents a group represented by Formula (q2), and Q³ represents a group represented by Formula (q3), 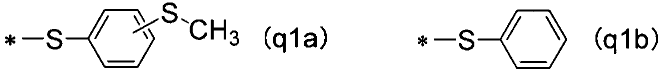 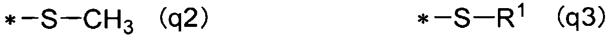
in the formulae, R¹ represents a (meth)acryloyl group, a vinyl group, or an allyl group, and * represents a bonding site.

2. The curable composition according to claim 1,
wherein the compound represented by General Formula (1) or (2) includes at least one of a compound in which substitution positions of the substituents (Q¹ and Q³) in General Formula (1) are (1,5) positions, (1,6) positions, (2,7) positions, (5,1) positions, (6,1) positions, or (7,2) positions of a naphthalene ring, or a compound in which substitution positions of the substituents (Q² and Q³) in General Formula (2) are (1,5) positions, (1,6) positions, (2,7) positions, (5,1) positions, (6,1) positions, or (7,2) positions of a naphthalene ring.

3. The curable composition according to claim 2,
wherein the compound represented by General Formula (1) or (2) includes at least one compound represented by any of General Formulae (3) to (6), 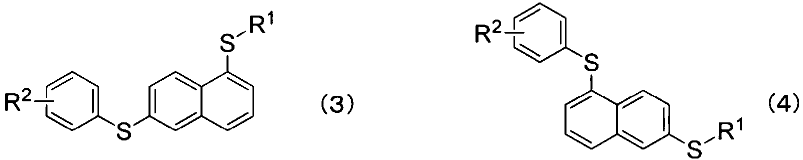 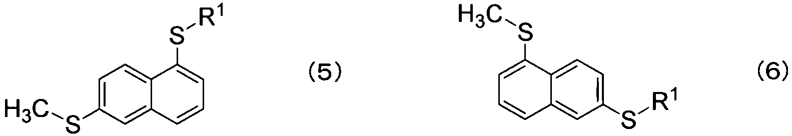
in the formulae, R¹ has the same definition as R¹ described above, and R² represents a hydrogen atom or a methylsulfanyl group.

4. The curable composition according to claim 1, further comprising:
a compound represented by General Formula (7), 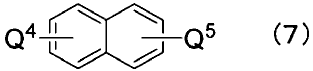
in the formula, Q⁴ and Q⁵ represent a group represented by any of Formula (qla), (qlb), (q2), or (q3), provided that one of Q⁴ and Q⁵ is a group represented by any of Formula (qla), (qlb), or (q2) and the other is not a group represented by Formula (q3), 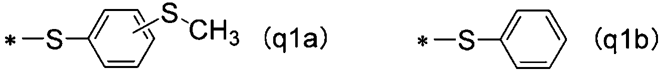 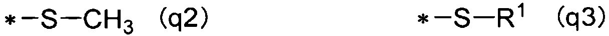
in the formulae, R¹ represents a (meth)acryloyl group, a vinyl group, or an allyl group, and * represents a bonding site.

5. The curable composition according to claim 1,
wherein the curable composition is for imprinting.

6. A cured product obtained from the curable composition according to any one of claims 1 to 5.

7. An optical material comprising:
the cured product according to claim 6.

8. A diffractive optical element which is formed of the cured product according to claim 6, comprising:
a surface having a diffraction grating shape.

9. A compound represented by General Formula (1) or (2), 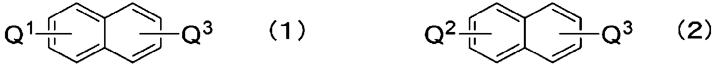
in the formulae, Q¹ represents a group represented by Formula (qla) or (qlb), Q² represents a group represented by Formula (q2), and Q³ represents a group represented by Formula (q3), 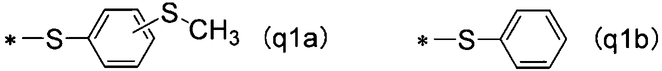 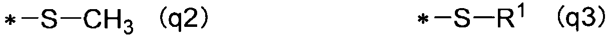
in the formulae, R¹ represents a (meth)acryloyl group, a vinyl group, or an allyl group, and * represents a bonding site.
